(19) Europäisches Patentamt / European Patent Office / Office européen des brevets

(11) **EP 1 733 205 B1**

(12) **FASCICULE DE BREVET EUROPEEN**

(45) Date de publication et mention
de la délivrance du brevet:
**06.07.2011 Bulletin 2011/27**

(51) Int Cl.:
***G01N 9/24*** *(2006.01)*        ***G21C 17/06*** *(2006.01)*

(21) Numéro de dépôt: **05753642.7**

(86) Numéro de dépôt international:
**PCT/FR2005/000838**

(22) Date de dépôt: **06.04.2005**

(87) Numéro de publication internationale:
**WO 2005/100952 (27.10.2005 Gazette 2005/43)**

(54) **PROCEDE ET SYSTEME DE DETERMINATION DE LA MASSE VOLUMIQUE ET DES CARACTERISTIQUES DIMENSIONNELLES D'UN OBJET, ET APPLICATION AU CONTROLE DES PASTILLES DE COMBUSTIBLE NUCLEAIRE EN COURS DE FABRICATION**

VERFAHREN UND SYSTEM ZUR BESTIMMUNG VON MASSEDICHTE UND ABMESSUNGSMERKMALEN EINES GEGENSTANDS SOWIE DESSEN VERWENDUNG ZUR ÜBERWACHUNG VON KERNBRENNSTOFFPELLETS WÄHREND DEREN HERSTELLUNG

METHOD AND SYSTEM FOR DETERMINING THE MASS DENSITY AND DIMENSIONAL CHARACTERISTICS OF AN OBJECT, AND USE THEREOF FOR CONTROLLING NUCLEAR FUEL PELLETS DURING THE PRODUCTION OF THE SAME

(84) Etats contractants désignés:
**AT BE BG CH CY CZ DE DK EE ES FI FR GB GR HU IE IS IT LI LT LU MC NL PL PT RO SE SI SK TR**

(30) Priorité: **06.04.2004  FR 0450686**

(43) Date de publication de la demande:
**20.12.2006 Bulletin 2006/51**

(73) Titulaires:
 • **Commissariat à l'Énergie Atomique et aux Énergies Alternatives**
   **75015 Paris (FR)**
 • **AREVA NC**
   **75009 Paris (FR)**

(72) Inventeurs:
 • **LYOUSSI, Abdallah**
   **04100 Manosque (FR)**

 • **PAYAN, Emmanuel,**
   **Rés. La Petite Négresse**
   **04100 Manosque (FR)**

(74) Mandataire: **Poulin, Gérard et al**
   **BREVALEX**
   **95 rue d'Amsterdam**
   **75378 Paris Cedex 8 (FR)**

(56) Documents cités:
   DE-A- 19 710 835     FR-A- 2 798 463
   US-A- 4 193 502      US-A- 6 151 379

 • FWA T F ET AL: "EXPERIMENTAL EVALUATION OF A LABORATORY TWIN-PROBE NUCLEAR GAGE FOR SPECIMEN DENSITY MEASUREMENT" JOURNAL OF TESTING AND EVALUATION, AMERICAN SOCIETY FOR TESTING AND MATERIALS. PHILADELPHIA, US, vol. 20, no. 1, janvier 1992 (1992-01), pages 59-65, XP000310735 ISSN: 0090-3973

**Description**

**DOMAINE TECHNIQUE**

La présente invention se rapporte au domaine des techniques d'analyse non destructives.

L'invention se rapporte plus précisément à un procédé et un système de détermination automatique de la masse volumique d'objets par atténuation photonique en ligne droite et de leurs caractéristiques dimensionnelles.

Elle trouve une application dans le contrôle et le suivi du bon fonctionnement d'unités de fabrication et d'usinage d'objets, comme par exemple des pastilles de combustible nucléaire, tel que UOX et/ou MOX, et permet en particulier de suivre la reproductibilité de fabrication desdits objets, en ce qui concerne leur masse volumique.

Elle peut aussi être utilisée pour déterminer des gradients axiaux est radiaux de masse volumique faisant ainsi office de tomodensitomètre très précis.

**ETAT DE LA TECHNIQUE ANTERIEURE**

Des méthodes nucléaires non destructives actives de détermination de la masse volumique ont déjà été élaborées, notamment pour déterminer la masse volumique d'échantillons géologiques. Dans le document référencé : Been, K., « Nondestructive Soil Bulk Density Measurement by X-ray Attenuation », Geotechnical Testing Journal, GTJODJ, Vol. 4, No. 4, Dec. 1981, pp 169-176, l'auteur propose une mesure de densité d'échantillons par atténuation photonique en ligne droite au moyen de tubes de rayons X, sans chercher à déterminer de façon précise la dimension des échantillons en question. Dans les documents référencés : Tan, S.-A. and Fwa, T. -F., « Nondestrcutive Density Measurements of Cylindrical Specimens by Gamma-Ray Attenuation », Journal of Testing Evaluation, JTEVA, Vol. 19, No. 2, March 1991, pp . 155-160, et : Tan, S. -A. and Fwa, T. -F. , « Density Measurements of Cylindrical Specimens within a Mold by Gamma-Rays », Journal of Testing Evaluation, JTEVA, Vol. 21, No. 4, July 1993, pp. 296-301, les auteurs proposent une mesure de densité d'échantillons géologiques par atténuation photonique en ligne droite, au moyen de rayonnement gamma. Ils ont identifié et montré l'impact des paramètres géométriques des échantillons sur la précision de la mesure de densité, sans pour autant proposer de solution pour déterminer avec précision lesdits paramètres géométriques.

On indique que, bien que les documents cités ci-dessus s'intéressent à la densité des échantillons, il s'agit en fait de la détermination de la masse volumique desdits objets, le terme « densité » étant utilisé à la place de « masse volumique » par facilité de langage.

**EXPOSÉ DE L'INVENTION**

La présente invention a pour but la détermination de la masse volumique d'objets appartenant à un lot donné d'objets, par détermination de la variation de la masse volumique de chacun desdits objets par rapport à la masse volumique, connue, de l'un au moins desdits objets pris comme référence ou étalon.

Cette détermination de la masse volumique desdits objets est effectuée au moyen d'une technique nucléaire non destructive, mettant en oeuvre un rayonnement de photons gamma, et d'une unité de détermination par spectrométrie gamma, de l'intensité du faisceau de photons gamma.

La détermination de la masse volumique desdits objets passe par la détermination préalable d'au moins une dimension significative de ceux-ci.

Contrairement aux méthodes développées dans l'art antérieur cité ci-dessus, l'invention tient compte de l'influence des paramètres géométriques, en mesurant de façon très précise au moins une dimension significative des objets dont on cherche à évaluer la masse volumique, et en utilisant cette dimension significative mesurée pour la détermination de la masse volumique des objets contrôlés. Ladite dimension significative peut être une largeur ou un diamètre, et correspond à la dimension effective traversée par le faisceau de photons gamma.

Le procédé de détermination de la dimension significative de l'objet fait partie du procédé de détermination de la masse volumique dudit objet. Il met en oeuvre une unité de mesure de dimension par rayonnement infrarouge.

On rappelle brièvement que le principe physique de la détermination de la masse volumique d'un objet par atténuation photonique consiste à irradier l'objet par un faisceau interrogateur de photons monochromatiques d'énergie E. L'intensité du faisceau photonique est plus ou moins atténuée en fonction de la masse volumique de l'objet traversé, de l'épaisseur de matière traversée, et de la composition chimique élémentaire de l'objet traversé. Cette intensité est fournie par l'équation :

$$I = I_o \exp(-\mu_m \rho\ x)$$

où :

- I est l'intensité atténuée du faisceau photonique, en photons$^{-1}$,
- $I_o$ est l'intensité non atténuée du faisceau photonique à l'énergie E, en photons$^{-1}$,
- $\mu_m$ est le coefficient massique d'atténuation du photon d'énergie E dans l'objet, en cm$^2$.g$^{-1}$,
- p est la masse volumique de l'objet à contrôler, en g.cm$^{-3}$,
- x est l'épaisseur de matière traversée par le faisceau photonique, ou dimension significative de l'objet, en cm.

L'expression de la masse volumique de l'objet s'en déduit directement par :

$$\rho = \frac{Ln\left[\dfrac{I_o}{I}\right]}{\mu_m x}$$

Ainsi la connaissance des intensités transmises avec et sans interposition de l'objet à contrôler, I et $I_o$ respectivement, du coefficient massique d'atténuation $\mu_m$ et de la dimension significative x de l'objet effectivement traversée permet de remonter à la masse volumique p dudit objet.

La présente invention se propose de déterminer l'épaisseur de matière x de l'objet traversée par le faisceau photonique et l'intensité transmise I du faisceau photonique au niveau d'énergie E, pour calculer ensuite la variation relative de la masse volumique p de cet objet par rapport à la masse volumique d'au moins un objet étalon pris comme référence. Une caractéristique de l'invention réside dans le fait que ces déterminations d'épaisseur de matière (dimension significative de l'objet) et d'intensité du faisceau photonique sont faites avec une précision micrométrique.

La variation relative de la masse volumique p de l'objet à contrôler est obtenue selon l'expression suivants :

$$\frac{\Delta\rho}{\rho} = \frac{\rho - \rho_e}{\rho} = \frac{x_e}{x}\left[1 - \frac{L_n \dfrac{I}{I_e}}{\mu_m \rho_e x_e}\right],$$

où $\rho_e$ est la masse volumique connue de l'objet pris comme étalon en masse volumique, et où $x_e$ est la dimension significative traversée de l'objet étalon en masse volumique.

Le coefficient massique d'atténuation $\mu_m$, qui dépend de la composition chimique de l'objet, est déterminé à partir d'un ou plusieurs objets étalons certifiés et parfaitement connus, et ayant la même composition chimique que celle de l'objet à contrôler. Il est déterminé lors d'une étape, qui sera décrite par la suite, d'étalonnage de l'unité de détermination de l'intensité du faisceau photonique, atténuée par la traversée de l'objet étalon.

Lorsque l'objet à contrôler est de section circulaire, la dimension significative traversée correspond à son diamètre. Lorsque l'objet à contrôler est de forme parallélépipédique, la dimension significative traversée correspond à une largeur de l'objet.

Par la suite on convient d'utiliser les notations suivantes lorsqu'il est nécessaire de distinguer un objet i du lot d'objets 100 et/ou un objet étalon e du lot d'objets 100 :

- l'indice emas est représentatif des grandeurs relatives à un objet étalon en masse volumique, comme par exemple sa dimension significative $x_{emas}$,
- l'indice edim est représentatif des grandeurs relatives à un objet étalon en dimension, comme par exemple sa dimension significative $x_{edim}$.

Selon un premier aspect de l'invention, le système pour la détermination automatique de la masse volumique d'un objet appartenant à un lot d'objets comprend :

- une unité de détermination d'une dimension significative dudit objet,
- une unité de détermination de l'intensité d'un faisceau photonique, atténuée par la traversée dudit objet,

- une unité d'acquisition, de traitement et d'analyse,
- des moyens de transport de l'objet vers l'unité de détermination de sa dimension significative et vers l'unité de détermination de l'intensité du faisceau photonique atténuée,
- des premiers moyens d'ajustement de la position de l'objet relativement à l'unité de détermination de la dimension significative,
- des deuxièmes moyens d'ajustement de la position de l'objet relativement à l'unité de détermination de l'intensité photonique atténuée,

lesdits premiers et deuxièmes moyens d'ajustement étant capables de déplacer l'objet avec une précision de l'ordre du micron par rapport à une plaque de support sur laquelle sont installés les éléments constitutifs du système, et la position de l'objet relativement à l'unité de détermination de l'intensité atténuée étant ajustée en fonction de la dimension significative dudit objet.

De préférence, l'unité de détermination d'une dimension significative de l'objet est une unité de mesure par rayonnement infrarouge.

De préférence, l'unité de détermination de l'intensité d'un faisceau photonique atténuée par la traversée de l'objet est une unité de détermination par spectrométrie gamma, qui comprend :

- un ensemble formé d'une source et d'un collimateur,
- un ensemble formé d'un détecteur et d'un collimateur,
- une chaîne d'acquisition et de comptage des photons gamma.

L'invention met en oeuvre des moyens de transport, ainsi que des moyens d'ajustement de position de chaque objet contrôlé par rapport à l'unité de détermination de la dimension significative de l'objet et/ou par rapport à l'unité de détermination de l'intensité atténuée, lesdits moyens d'ajustement de position étant capables de fournir une précision de l'ordre du micron.

Selon un deuxième aspect, l'invention concerne un procédé de mise en oeuvre du système pour la détermination automatique de la masse volumique d'un objet (100) appartenant à un lot d'objets, comprend les étapes d'étalonnage suivantes :

- une étape 1 d'étalonnage de position de deux ensembles infrarouges de l'unité de détermination de la dimension significative des objets,
- une étape 2 d'étalonnage de position d'un support d'irradiation de l'unité de détermination par spectrométrie gamma de l'intensité du faisceau photonique atténuée par la traversée des objets,
- une étape 3 d'étalonnage de mesure d'un ensemble source-détecteur de l'unité de détermination par spectrométrie gamma de l'intensité du faisceau photonique atténuée par la traversée des objets,

et il comprend des étapes de détermination proprement dite de la dimension significative des objets, qui sont effectuées sur chaque objet dudit lot d'objets.

Selon l'invention, les étapes de détermination proprement dite comprennent :

- une étape 4 de détermination de la dimension significative de l'objet à contrôler,
- une étape 5 de transport de l'objet vers un support d'irradiation,
- une étape 6 d'ajustement de la position de l'objet par ajustement de la position du support d'irradiation par rapport à une source et un détecteur associé,
- une étape 7 de détermination de l'intensité atténuée du faisceau photonique transmis à travers l'objet,
- une étape 8 d'acquisition, traitement et analyse du spectre obtenu,
- une étape 9 de détermination de la variation relative $\dfrac{\Delta\rho}{\rho}$ de masse volumique de l'objet par rapport à celle d'un ou plusieurs objet(s) étalon(s) en masse volumique,
- une étape 10 de transport retour de l'objet jusqu'à son emplacement sur le plateau tournant.

Les procédés et dispositifs selon l'invention présentent l'intérêt commun d'être rapides, précis, automatiques ou automatisables, et d'une utilisation aisée.

Un avantage de l'invention réside dans le fait qu'elle associe l'atténuation photonique en ligne droite avec une métrologie micrométrique afin de pallier les incertitudes liées à la méconnaissance des épaisseurs des objets traversés, et qui affectent directement la précision de la détermination de la masse volumique.

En particulier, le positionnement de chaque objet par rapport à l'unité de détermination de l'intensité photonique atténuée par la traversée dudit objet est ajusté en fonction d'une dimension significative dudit objet, qui a été préalablement déterminée par l'unité de détermination de dimension significative.

**BRÈVE DESCRIPTION DES DESSINS**

L'invention sera mieux comprise à la lecture de la description détaillée qui va suivre d'un mode de réalisation préféré, donné à titre d'exemple non limitatif, qui est illustré par les dessins annexés dans lesquels :

- la figure 1 est une vue schématique de dessus du système général de détermination de la dimension significative et de la détermination de la masse volumique des objets,
- la figure 2 est une vue schématique en perspective du système général de détermination de la dimension significative et de la détermination de masse volumique des objets,
- les figures 3, 4, et 5 illustrent en vue de dessus et de façon schématique le dispositif de détermination d'une dimension significative des objets au moyen de rayonnement infrarouge, et trois phases du procédé de détermination de cette dimension significative,

- la figure 6 est une vue en perspective et en coupe du collimateur de la source d'irradiation photonique,
- la figure 7 est une vue en perspective et en coupe du collimateur du détecteur des photons gamma,
- la figure 8 est une vue schématique montrant la chaîne d'acquisition et de comptage,
- les figures 9A et 9B illustrent l'ensemble des étapes du procédé de détermination de la masse volumique des objets ; la figure 9A illustre les étapes d'étalonnage préalables et la figure 9B illustre les étapes de détermination proprement dites ;
- la figure 10 illustre la première étape du procédé, qui est une étape d'étalonnage de position de l'unité de détermination de la dimension significative ;
- la figure 11 illustre la deuxième étape du procédé, qui est une étape d'étalonnage de position de l'unité de détermination de l'intensité photonique atténuée ;

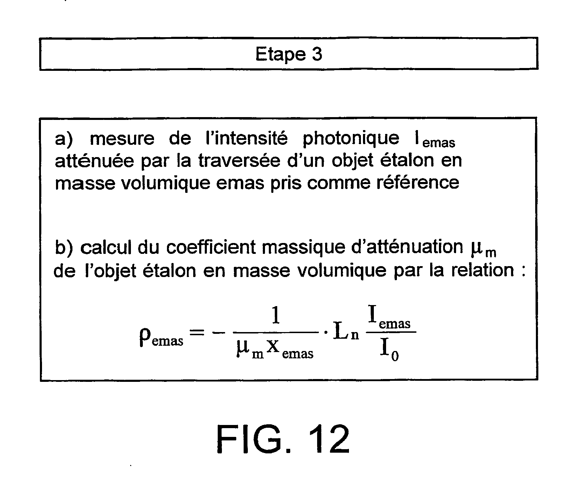

- la figure 12 illustre la troisième étape du procédé, qui est une étape d'étalonnage de mesure de l'unité de détermination de l'intensité photonique atténué
- la figure 13 illustre la quatrième étape du procédé, qui est une étape de détermination de la dimension significative d'un objet ;
- la figure 14 illustre la neuvième étape du procédé, qui est une étape de détermination de la variation relative de masse volumique de l'objet par rapport à celle d'un ou plusieurs objets étalons ;
- la figure 15 est un graphique illustrant la variation relative de masse volumique des objets d'un lot donné d'objets par rapport à la masse volumique de l'un des objets pris comme étalon ou référence, et compare cette variation relative de masse volumique obtenue par l'invention avec la variation relative de masse volumique théorique fournie par le fabriquant d'objets.

**EXPOSÉ DÉTAILLÉ D'UN MODE DE RÉALISATION PARTICULIER**

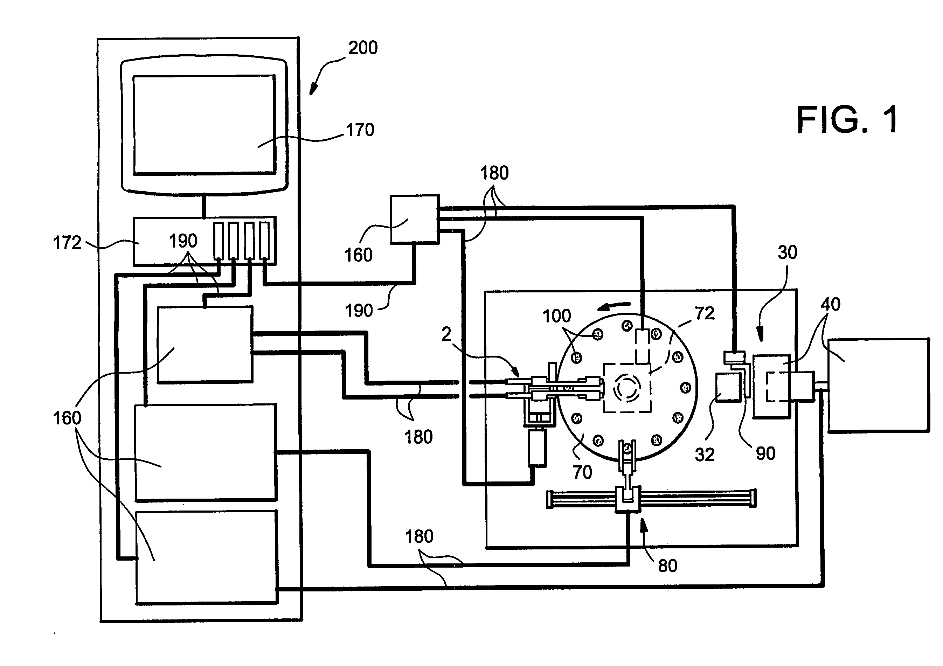

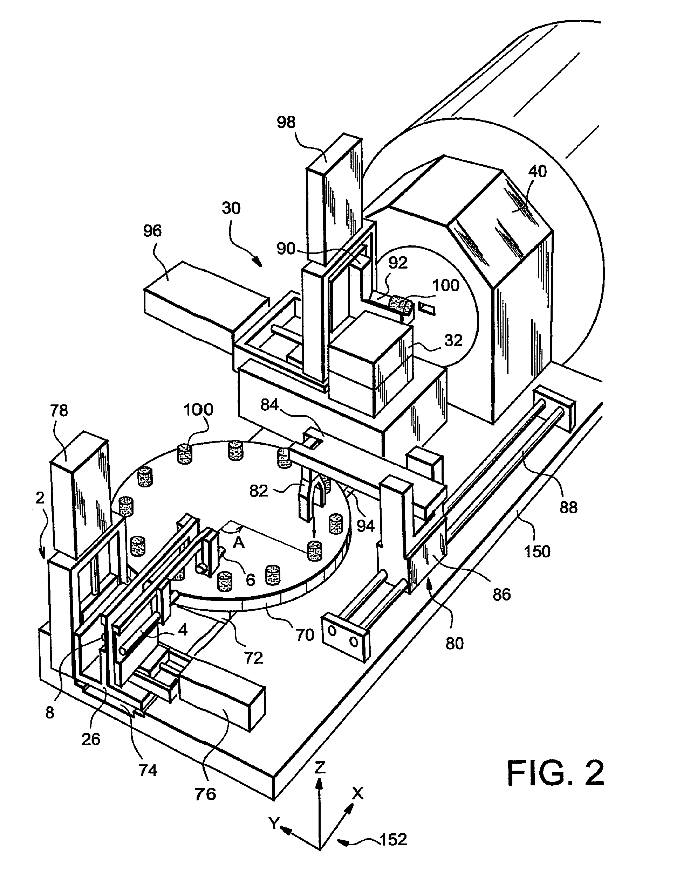

Les figures 1 et 2 illustrent respectivement en vue de dessus et en perspective un mode de réalisation préféré du système général de détermination par atténuation photonique de la masse volumique de chaque objet 100 d'un lot d'objets par détermination de la variation relative de cette masse volumique par rapport à celle d'au moins un desdits objets pris comme référence ou étalon en masse volumique, cette détermination de masse volumique mettant en oeuvre la détermination préalable d'une dimension significative x dudit objet 100, et de l'intensité I du faisceau photonique qui irradie et traverse ledit objet 100.

Le système comprend les composants suivants :

- une unité 2 de détermination de la dimension significative de l'objet 100,
- une unité 30 de détermination de l'intensité du faisceau photonique atténué par la traversée de l'objet 100,
- une unité 200 d'acquisition, de traitement, et d'analyse.
- des moyens de transport 70, 72, 80, 82, 84, 86, 88 et des moyens d'ajustement de position 74, 76, 78, 90, 92, 94, 96, 98 de l'objet 100, respectivement par rapport à l'unité 2 de détermination de dimension et par rapport à l'unité 30 de détermination d'intensité,

L'unité 200 d'acquisition, de traitement et d'analyse est schématisée dans son ensemble à la figure 1. Elle comprend notamment un ordinateur 170 de type PC sur lequel est implanté un logiciel dédié qui exécute des séries d'instructions et des algorithmes de calcul du procédé de détermination automatique de la masse volumique d'objets 100 selon l'invention.

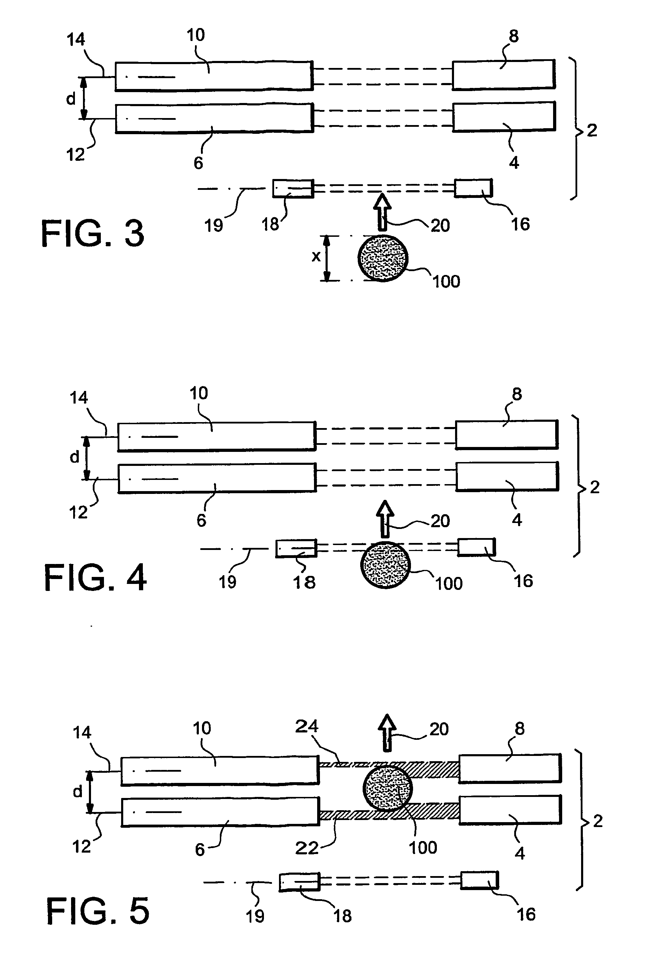

En se référant aux figures 3, 4, et 5, l'unité 2 de détermination par rayonnement infrarouge d'une dimension significative x de l'objet 100 comprend :

- un premier ensemble infrarouge 4, 6 constitué d'un premier émetteur infrarouge 4 et d'un premier récepteur infrarouge 6,
- un deuxième ensemble infrarouge 8, 10 constitué d'un deuxième émetteur infrarouge 8 et d'un deuxième récepteur infrarouge 10.

Les deux ensembles infrarouges 4, 6, et 8, 10, sont agencés de telle manière que les axes respectifs 12, 14 des faisceaux de rayonnement infrarouges qu'ils engendrent sont parallèles entre eux, et écartés d'une distance d. Cette distance d, fixée par le constructeur, est choisie de manière à être du même ordre de grandeur que la dimension significative x des objets 100 à contrôler. Elle est ajustable. Sur l'exemple illustré, les faisceaux infrarouges sont orientés dans le même sens, mais une configuration différente pourrait être envisagée.

L'unité 2 de détermination de la dimension significative x de l'objet 100 par rayonnement infrarouge comprend en outre un troisième ensemble constitué d'un émetteur photoélectrique 16 et d'un récepteur photoélectrique 18, disposé en amont du premier ensemble infrarouge 4, 6, par rapport au deuxième ensemble infrarouge 8, 10, le faisceau photoélectrique qu'il engendre ayant un axe 19. Sur l'exemple illustré, l'axe 19 du faisceau photoélectrique est parallèle aux axes 12, 14 des faisceaux infrarouges, et situé dans le même plan que ceux-ci. Une configuration différente pourrait être envisagée.

L'unité 2 de détermination de la dimension significative x de l'objet 100 par rayonnement infrarouge est associée à des moyens de transport et/ou à des moyens d'ajustement de position de l'objet 100 par rapport aux trois ensembles émetteurs-récepteurs 4, 6, 8, 10, 16, 18, qui seront décrits par la suite.

En fonctionnement, l'unité 2 de détermination de la dimension significative de l'objet 100 se trouve dans une situation dans laquelle les trois ensembles émetteurs-récepteurs 4, 6, 8, 10, 16, 18 sont fixes, et l'objet 100 est déplacé de manière à intercepter successivement le faisceau photoélectrique, puis le premier faisceau infrarouge, puis le deuxième faisceau infrarouge.

L'unité 2 est étalonnée de manière à établir une distance d entre les axes 12 et 14 des deux faisceaux de rayonnement infrarouge qui soit sensiblement proche de la dimension significative $x_{edim}$ d'un ou plusieurs objets edim pris comme étalons en dimension. Cet étalonnage sera décrit par la suite. Il s'ensuit que lors de la détermination de la dimension significative d'un objet 100 (non étalon), celui-ci se déplace relativement aux trois ensembles émetteurs-récepteurs 4, 6, 8, 10, 16, 18, et passe par au moins une position dans laquelle il intercepte encore la moitié du premier faisceau infrarouge (figure 5, référence 22), et n'intercepte pas encore la totalité du deuxième faisceau infrarouge, laissant une fraction du deuxième faisceau (figure 5, référence 24) qui n'est pas interceptée par l'objet 100, et qui atteint le deuxième récepteur 10.

La dimension significative x de l'objet 100 se déduit de la réponse infrarouge RI correspondant à cette fraction de faisceau non interceptée. Cette dimension est obtenue par une relation du type : $x = A_4.(RI^4) + A_3.(RI^3) + A_2.(RI^2) + A_1.(RI^1) + A_0$, où $A_4, A_3, A_2, A_1, A_0$ sont des coefficients obtenus en utilisant au moins quatre objets étalons en dimension edim, et en appliquant autant de fois cette même relation, dans laquelle sont injectées la dimension significative $x_{edim}$ connue et la réponse infrarouge $RI_{edim}$ mesurée de chacun desdits objets étalons en dimension edim.

Comme représenté sur la figure 4, le troisième ensemble 16, 18 a pour fonction de déclencher automatiquement l'ajustement préalable de l'intensité des deux faisceaux infrarouges 22, 24, lorsqu'au cours du déplacement relatif selon la direction 20, l'objet 100 intercepte le faisceau photoélectrique engendré par ce troisième ensemble 16, 18. Cette opération a pour but de s'affranchir des perturbations environnementales telles que la salissure des optiques. Elle doit avoir lieu au plus tard 30 secondes avant l'opération de mesure proprement dite sur l'objet 100.

La précision de la détermination de la dimension significative x de l'objet 100 est fonction de la précision du déplacement relatif de l'objet 100 par rapport aux trois ensembles émetteurs-récepteurs 4, 6, 8, 10, 16, 18, et donc des performances et de l'étalonnage des moyens de transport et/ou d'ajustement de position, aspects qui seront décrits plus en détail par la suite.

Selon l'invention, la détermination de l'intensité I du faisceau photonique est effectuée au moyen d'une unité 30 de détermination par spectrométrie gamma de l'intensité du faisceau photonique qui irradie et traverse les objets 100, comme illustré aux figures 1, 2 et 8. Celle-ci comprend :

- un ensemble formé d'une source d'irradiation photonique et d'un collimateur 32, de type connu en soi,
- un ensemble formé d'un détecteur et d'un collimateur 40, de type connu en soi,
- une chaîne d'acquisition et de comptage 48, de type connu en soi.

Pour simplifier la description, la source d'irradiation photonique sera par la suite nommée "source".

Les différents éléments constitutifs de l'unité 30 de détermination sont soumis à certaines contraintes liées à la performance souhaitée pour le système général, ainsi qu'à l'environnement dans lequel le système est destiné à fonctionner. Ces contraintes, qui concernent notamment l'intensité de la source, le type de source, et les performances de la chaîne d'acquisition et de comptage, sont les suivantes :

- la source doit avoir une intensité telle que la dispersion statistique des résultats de mesure soit nettement inférieure à la variation de comptage due à un écart de masse volumique de l'objet à contrôler par rapport à la masse volumique de l'objet étalon pris comme référence,
- l'énergie de la source doit permettre un très bon contraste à la suite d'une légère variation de la masse volumique de l'objet à contrôler,
- la source ne doit pas présenter une période radioactive trop courte de manière à ne pas être contraignante en milieu industriel,
- enfin, l'intensité et l'énergie de la source doivent être compatibles avec les capacités de traitement de la chaîne électronique d'acquisition et de comptage (temps mort, empilement, saturation...).

Selon le mode de réalisation préféré, la source est réalisée en $^{133}$Ba d'au moins 10 mCi d'activité. Pour éviter les effets de temps mort et/ou de saturation, il est préféré d'utiliser une source dont l'activité ne dépasse pas 150 mCi. La durée de mesure est inversement proportionnelle à l'activité de la source.

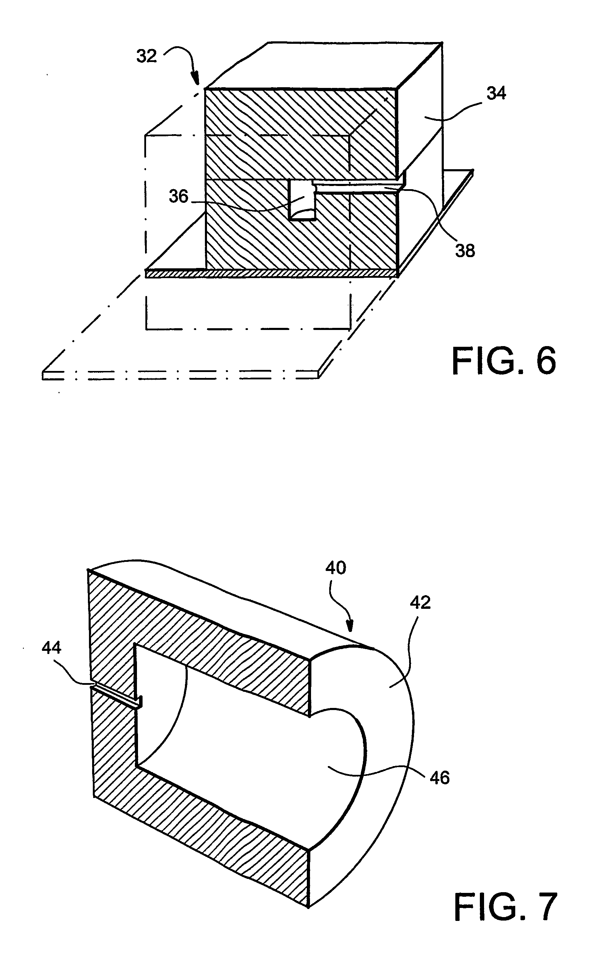

Un exemple de réalisation du collimateur 32 de l'ensemble source-collimateur, compatible avec ces différentes contraintes, est illustré à la figure 6. Il comprend un coffrage de protection 34, pour assurer la sécurité des personnes travaillant à proximité de la source, qui délimite une cavité 36 dans laquelle est logée la source. Le faisceau de photons gamma est guidé par une fente de collimation 38.

Selon l'exemple de réalisation illustré, le collimateur 32 de la source est réalisé en plomb et présente comme dimensions extérieures une hauteur de 60 mm, une longueur de 60 mm, et une largeur de 60 mm. La source est une source de $^{133}$Ba de 10 mCi d'activité, qui est logée dans une cavité 36 ayant un diamètre de 6,1 mm et une hauteur de 9,5 mm. La fente de collimation 38 présente quant à elle une longueur de 30 mm, une largeur de 6 mm, et une hauteur de 4 mm.

Un exemple de réalisation du collimateur 40 de l'ensemble détecteur-collimateur est illustré à la figure 7. Il comprend un coffrage de protection 42 pour que les rayons gamma, issus de la source et émis en dehors de la fente de collimation 38 ne soient pas perçus par le détecteur 49, une fente de collimation 44, et une cavité 46 de logement du détecteur 49, délimitée par le coffrage de protection 42.

Selon l'exemple de réalisation illustré, le collimateur 40 du détecteur 49 est réalisé en plomb et présente comme dimensions extérieures un diamètre de 140 mm et une longueur de 120 mm, et comme dimensions intérieures un diamètre de 80 mm et une longueur de 200 mm. La fente de collimation présente une hauteur de 4 mm, une largeur de 6 mm, et une longueur de 30 mm.

Le plomb du coffrage de protection 42 peut être remplacé par du tungstène, qui atténue davantage les rayons gamma que le plomb, ce qui présente l'avantage de réduire l'épaisseur du coffrage de protection 42, mais le tungstène a pour inconvénient son prix plus élevé que celui du plomb.

Par la suite, et pour simplifier la description, l'ensemble source-collimateur sera dénommé simplement « source » et référencé 32, et l'ensemble détecteur-collimateur sera dénommé simplement « détecteur » et sera référencé 40.

La distance source-détecteur est choisie de façon appropriée.

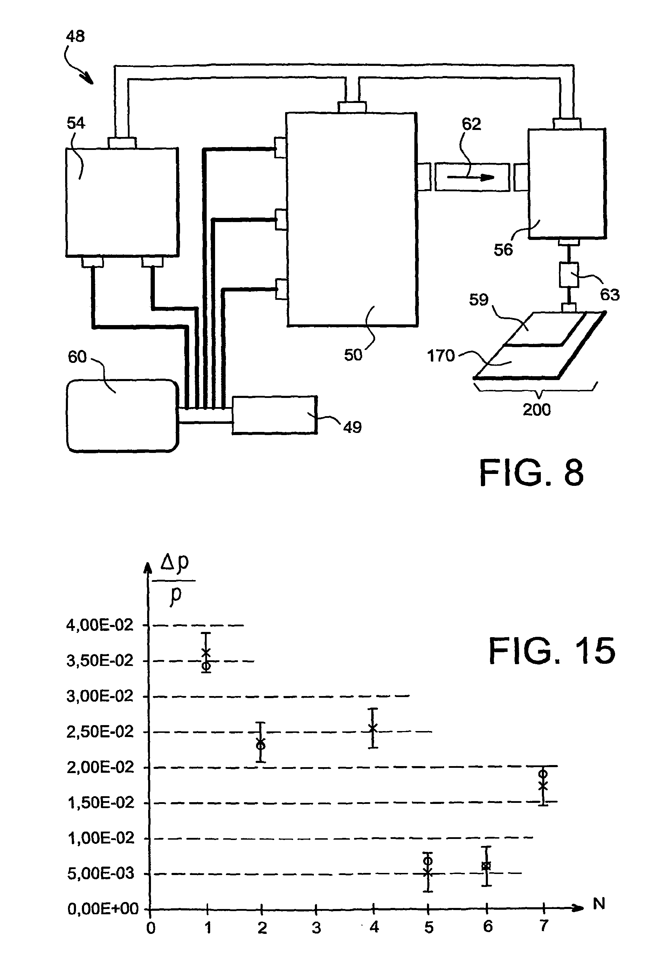

Selon le mode de réalisation préféré, la chaîne d'acquisition et de comptage 48, illustrée à la figure 8, comprend :

- un détecteur 49, sous forme d'une diode à germanium de haute pureté Ge [HP], ayant un préamplificateur,
- un module 50 de traitement du signal (DSP : Digital Signal Processor),
- un module haute tension 54,
- un module réseau 56 d'acquisition et d'interface (AIM : acquisition interface module),
- un ordinateur 170 de type PC d'acquisition de données (figure 1).

De façon optionnelle la chaîne d'acquisition et de comptage comprend un cryostat 60 constitué d'un réservoir d'azote liquide, qui maintient à température constante le doigt froid de la diode Ge [HP], ce qui présente l'avantage de minimiser l'effet Doppler et d'obtenir une très bonne résolution du signal, les mesures n'étant pas perturbées par un échauffement du détecteur 49.

Le préamplificateur est de préférence incorporé dans la diode Ge [HP], ce qui présente l'avantage de minimiser l'effet de capacité dû au câble électrique et de réduire le bruit de fond électronique. Il assure également le filtrage et la mise en forme du signal.

Le signal est ensuite numérisé au moyen du module de traitement du signal 50, puis mis en mémoire.

L'ensemble des informations obtenus constitue le spectre gamma, c'est à dire l'histogramme classant dans différents canaux le nombre d'impulsions en fonction de leur énergie.

Les données sont transférées (flèche 62) entre le module 50 de traitement du signal et l'ordinateur 170 de l'unité 200, d'acquisition, de traitement et d'analyse, via le module réseau 56 d'acquisition et d'interface, un émetteur-récepteur 63, et une carte réseau 59. Sur l'exemple illustré, l'unité 200 d'acquisition, de traitement et d'analyse ainsi que la chaîne 48 d'acquisition et de comptage utilisent le même ordinateur 170, mais une configuration avec deux ordinateurs distincts pourrait être envisagée.

Cette chaîne d'acquisition et de comptage 48 est particulièrement adaptée aux forts taux de comptage.

Par ailleurs, une autre contrainte d'utilisation de l'unité 30 de détermination par spectrométrie gamma de l'intensité du faisceau photonique qui irradie les objets 100 concerne le temps de comptage de la chaîne 48 d'acquisition et de comptage, qui doit respecter les cadences de fabrication des objets 100 à contrôler.

Selon l'invention, le temps de comptage peut être une donnée d'entrée du système, ou bien le résultat d'un calcul, fourni par la relation théorique suivante :

$$t = \frac{\alpha^2}{A(t) \cdot \frac{s}{4\pi D^2} \cdot \varepsilon \cdot \frac{\sum}{P} \cdot R_0 \cdot \left(R_0^{\beta_{sec}} - 1\right)^2}$$

avec une approximation selon laquelle l'angle solide est égal à $4\pi D^2$.
où :

A(t) est l'activité de la source, en Bq,
D est la distance entre la source et une fenêtre de collimation, en mm,
s est la surface de la fenêtre de collimation du détecteur, en mm$^2$,
$\alpha$ est la largeur de l'intervalle de confiance dans l'hypothèse où le comptage suit une loi de Poisson,
e est l'efficacité d'absorption totale du détecteur de photons,
I est l'intensité du faisceau photonique à l'énergie E, atténuée par la traversée de l'objet, en $\gamma.s^{-1}$,
$I_0$ est l'intensité non atténuée du faisceau photonique à l'énergie E, en $\gamma. s^{-1}$,

$R_0 = \frac{I}{I_0}$ est le coefficient de transmission de l'objet traversé par les photons monochromatiques issus de la source,

$\Sigma$ est le nombre total de coups enregistrés dans le spectre mesuré, en coups,
P est le nombre total de coups contenus dans le pic d'énergie E,

$\beta_{sec} = \frac{\beta}{10}$ est une valeur de $\beta$ affectée d'un facteur de sécurité égal à 10,

avec $\beta = \frac{\Delta\rho}{\rho}$,

et où p est la masse volumique de l'objet.

La précision de la détermination de l'intensité I atténuée par la traversée de l'objet 100 dépend notamment de la position dudit objet 100 par rapport à la source 32. Elle dépend donc des performances et de l'étalonnage des moyens d'ajustement de position. Ces aspects seront décrits plus en détail par la suite.

Les différents moyens de transport 70, 72, 80, 82, 84, 86, 88 et d'ajustement de position 74, 76, 78, 90, 92, 94, 96, 98 sont illustrés aux figures 1 et 2 montrant le système dans son ensemble. Ils ont pour fonction de transporter l'objet 100 vers chaque unité 2, 30 de détermination ou d'ajuster la position relative de l'objet 100 par rapport aux éléments constitutifs de chaque unité 2, 30 de détermination.

Une plaque de support 150 supporte les composants du système général, à savoir l'unité 2 de détermination de la dimension significative, l'unité 30 de détermination de l'intensité du faisceau atténué, les moyens de transport, les premiers moyens d'ajustement, et les deuxièmes moyens d'ajustement. Les directions de déplacement sont schématisées par le référentiel 152 de la figure 2. Les déplacements ont lieu dans le plan horizontal (X, Y) de la plaque de support 150, ou selon la direction verticale Z perpendiculaire au plan horizontal (X, Y) de la plaque de support 150.

Les moyens de transport 70, 72, ont pour fonction de transporter l'objet 100 dans une première position dans laquelle l'unité 2 détermine la dimension significative dudit objet 100. Ils comprennent un plateau tournant horizontal 70 actionné par un moteur pas à pas 72, tous deux installés sur la plaque de support 150. Sur l'exemple illustré, le plateau tournant 70 comprend douze emplacements d'objets.

Les premiers moyens d'ajustement 74, 76, 78 ont pour fonction d'ajuster la position de l'objet 100 par rapport aux deux ensembles infrarouges 4, 6 et 8, 10, au moyen desquels est mesurée la dimension significative x de l'objet 100.

Le moyen d'ajustement 74 est une glissière orientée suivant la direction X, dans laquelle sont positionnés le socle 26 de l'unité 2 de détermination de dimension par rayonnement infrarouge, et le plateau tournant 70.

Les deux ensembles infrarouges 4, 6, et 8, 10 sont installés sur le socle 26 de telle manière que les axes 12, 14 des faisceaux infrarouges soient parallèles à cette direction X. Pour un lot donné d'objets dont les dimensions sont sensiblement toutes du même ordre de grandeur, les positions relatives du socle 26 et du plateau tournant 70 selon cette direction X sont de préférence fixés une fois pour toutes au début de la série de mesures pour le lot donné d'objets.

Le moyen d'ajustement 76 est un vérin dont la fonction est de rapprocher ou écarter le premier ensemble infrarouge. 4, 6 du deuxième ensemble infrarouge 8, 10 suivant la direction Y. Ce déplacement du premier ensemble infrarouge 4, 6 selon la direction Y permet de positionner avec une précision micrométrique l'objet 100 par rapport aux deux faisceaux de rayonnement infrarouge pour la détermination de sa dimension significative x, diamètre ou épaisseur.

Le moyen d'ajustement 78 est un vérin dont la fonction est de déplacer le socle 26 suivant la direction Z. L'amplitude de ce déplacement est relativement faible, afin d'éviter que le socle 26 sorte de la glissière 74. Le déplacement du socle selon la direction Z permet de connaître avec une précision micrométrique la cote de l'objet 100 à laquelle est effectuée la détermination de sa dimension significative x.

Les moyens de transport 70, 72, 80, 82, 84, 86, 88 ont également pour fonction de déplacer l'objet 100 de sa première position dans laquelle l'unité 2 détermine la dimension significative x vers sa deuxième position dans laquelle l'unité 30 détermine l'intensité atténuée I du faisceau photonique. Ils comprennent le plateau tournant 70 actionné par son moteur pas à pas 72. En effet, plusieurs objets 100 étant disposés en cercle sur le plateau tournant 70, la rotation dudit plateau 70 remplit deux actions simultanées consistant d'une part à transporter un objet 100 vers sa première position de mesure et d'autre part à éloigner l'objet 100 précédent de sa première position de mesure pour l'emmener vers une position intermédiaire après lui avoir fait effectuer un déplacement angulaire d'angle A. Sur l'exemple illustré aux figures 1 et 2, cet angle A est de 90°. Les moyens de transport comprennent également un bras de manutention 80 qui saisit l'objet 100 installé sur le plateau tournant 70 en sa position intermédiaire et le transporte sur un support d'irradiation 90 disposé entre le collimateur 32 de la source et le collimateur 40 du détecteur. Selon l'exemple illustré à la figure 2, le bras de manutention 80 comprend une pince de préhension 82 articulée sur un segment intermédiaire 84, lui-même articulé sur un vérin 86 capable de se déplacer en translation selon la direction X de la plaque de support 150, le long de rails de guidage 88. Les mouvements de serrage/desserrage de la pince 82 et de pivotement de celle-ci par rapport au segment 84, ainsi que les mouvements de pivotement du segment 84 par rapport au vérin 86 sont commandés par des actionneurs (non représentées).

Les deuxièmes moyens d'ajustement 90, 92, 94, 96, 98 ont pour fonction d'ajuster la position de l'objet 100 par rapport à la source 32 et au détecteur 40 de l'unité 30 de détermination par spectrométrie gamma de l'intensité du faisceau qui va traverser ledit objet 100. Ils comprennent le support d'irradiation 90 sur lequel est installé l'objet 100. Ce support d'irradiation 90 présente une face supérieure 92 à section en forme de V, ou tout autre moyen équivalent pour que l'objet 100 se trouve automatiquement installé en position d'équilibre stable sur ledit support d'irradiation 90, et notamment qu'il soit empêché de se déplacer par rapport au support d'irradiation 90 selon la direction X de la plaque de support 150. Le support d'irradiation 90 est positionné suivant la direction X de la plaque de support 150 au moyen d'une glissière 94, qui, de préférence, est confondue avec la glissière 74. Pour un lot donné d'objets 100, ce positionnement est effectué une fois pour toutes au début de la série de mesures correspondant à un lot donné d'objets. Le support d'irradiation 90 peut être déplacé selon la direction Y de la plaque de support 150 au moyen d'un vérin 96 et selon la direction Z perpendiculaire à la plaque de support 150 au moyen d'un vérin 98. Les ajustements de position effectués par les vérins 96 et 98 permettent de centrer sensiblement l'objet (suivant la direction Z) entre les fentes des collimateurs respectifs de la source 32 et du détecteur 40.

D'autre part il est nécessaire de positionner avec une précision micrométrique l'objet suivant la direction Y de telle manière que la mesure d'intensité I du faisceau photonique s'effectue exactement à la cote de l'objet à laquelle a été effectuée la détermination de sa dimension significative x. Ce positionnement est effectué par une mise en butée de l'objet sur la face supérieure 92 du support d'irradiation 90. Cette mise en butée peut être effectuée par exemple par une opération de soufflage au moyen d'un dispositif de soufflage (non représenté, qui envoie de l'air comprimé sur l'objet, suivant la direction Y, afin de plaquer celui-ci contre une butée 93 du support d'irradiation 90.

Sur la figure 1. sont illustrées les liaisons par des moyens de connexion appropriés 180 entre d'une part les différents vérins 76, 78, 86, 96, 98 de déplacement des pièces mobiles en translation et le moteur pas à pas 72 qui entraîne en rotation le plateau tournant 70, et d'autre part des unités 160 de contrôle et de pilotage. Ces unités 160 commandent la mécanique et l'automatique du système, et sont reliées à l'unité centrale 172 de l'ordinateur 170 de l'unité 200 d'acquisition, de traitement et d'analyse, par d'autres moyens de connexion appropriés 190.

On va maintenant décrire le procédé de détermination de la masse volumique p de chacun des objets d'un lot donné d'objets 100, par comparaison avec la masse volumique $\rho_{emas}$ d'un ou plusieurs objet(s) choisi (s) comme étalon (s) ou référence(s) en masse volumique, et faisant partie du même lot d'objets 100

Le procédé est mis en oeuvre au moyen d'algorithmes traduisant des séries d'instructions qui permettent d'effectuer de manière automatique les différentes étapes du procédé.

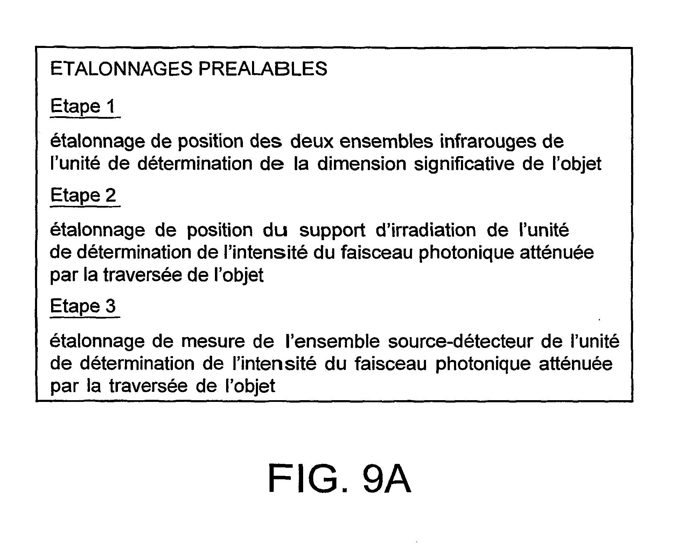

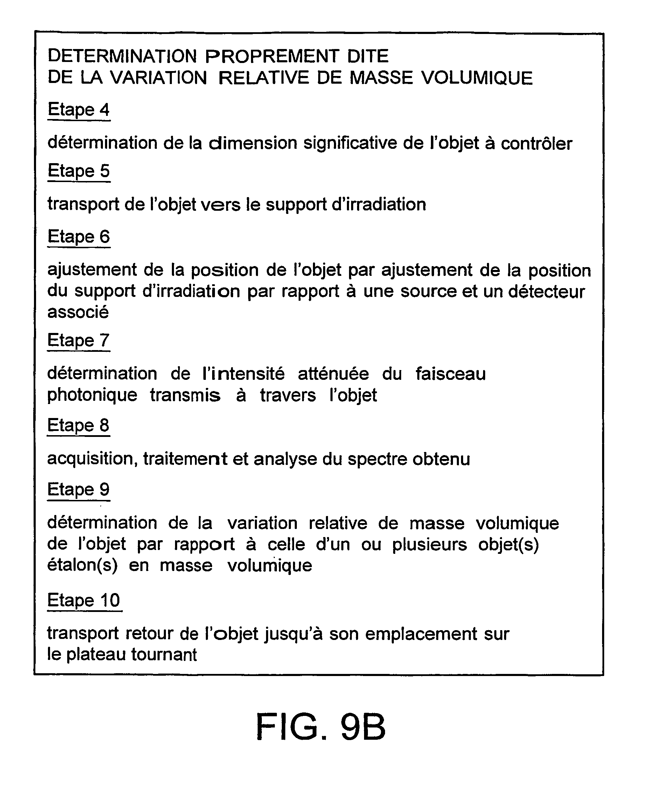

Le procédé de l'invention comprend des étapes préalables d'étalonnage, qui sont effectuées une fois pour toutes avant de commencer une série de mesures sur un lot donné d'objets, et des étapes de détermination proprement dites, qui sont effectuées sur chaque objet 100 dudit lot d'objets. L'ensemble des étapes du procédé est représenté de façon synthétique sur les figures 9A et 9B.

Les étapes d'étalonnage respectent une chronologie pré-établie, et concernent les composants suivants du système :

- étape 1 : étalonnage de position des deux ensembles infrarouges 4, 6 et 8, 10 de l'unité 2 de détermination de la dimension significative des objets 100,
  étape 2 : étalonnage de position du support d'irradiation 90 de l'unité 30 de détermination par spectrométrie gamma de l'intensité du faisceau photonique atténuée par la traversée des objets 100,
- étape 3 : étalonnage de mesure de l'ensemble source-détecteur 32, 40 de l'unité 30.

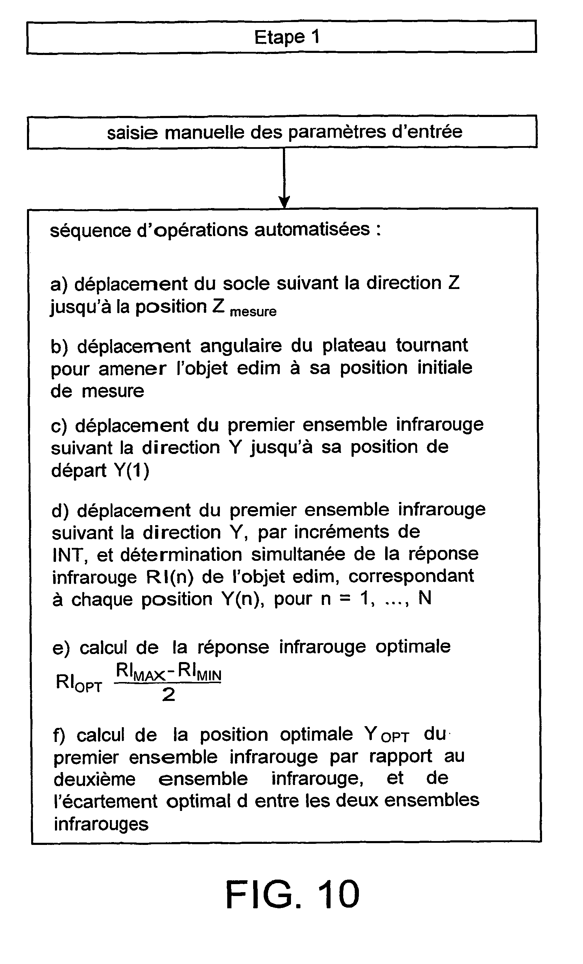

L'étape 1 d'étalonnage de position des deux ensembles infrarouges 4, 6 et 8, 10 est illustrée sur la figure 10.

Cette étape 1 d'étalonnage consiste à ajuster la position suivant la direction Y du premier ensemble infrarouge 4, 6 par rapport au deuxième ensemble infrarouge 8, 10, afin de fixer l'écartement d entre les faisceaux infrarouges émis respectivement par les deux émetteurs 4, 8, en fonction de la dimension significative $x_{edim}$ connue précisément d'un ou plusieurs objet(s) étalon(s) en dimension edim. En pratique, l'écartement d est établi en éloignant progressivement le premier ensemble infrarouge 4, 6 du deuxième ensemble infrarouge 8, 10 suivant la direction Y, ce dernier restant fixé à une position $Y_{FIX}$, et en mesurant la réponse infrarouge de l'objet pour chaque position du premier ensemble infrarouge 4, 6.

L'étape 1 d'étalonnage de position des deux ensembles infrarouges 4, 6 et 8, 10 comprend tout d'abord une saisie par l'opérateur d'un ensemble de paramètres d'entrée au moyen d'un module interactif. Ces paramètres comprennent :

- la configuration des composants qui ont un déplacement micrométrique : vérins 76, 78, ce qui permet de gérer leur cinématique : position, vitesse, accélération,
- la configuration du plateau tournant 70, c'est-à-dire la nature des objets qui occupent les différents emplacements sur le plateau tournant 70 : objet quelconque 100, ou objet étalon en dimension edim, ou objet étalon en masse volumique emas, ou emplacement libre,
- l'emplacement occupé par les objets étalons en dimension edim sur le plateau tournant 70, cet emplacement étant un nombre allant de 1 à 12 pour l'exemple illustré,

- la position $Z_{mesure}$ suivant la direction Z du socle 26 de l'unité 2, qui correspond à une cote $z_{edim}$ sur l'objet edim par rapport à la base de celui-ci,
- les positions Y(1) et Y(N) bornant l'intervalle de déplacement du premier ensemble infrarouge 4, 6 suivant la direction Y,

- le pas INT exprimé en $\mu$m du déplacement du premier ensemble infrarouge 4, 6 suivant la direction Y, (il faut que

$$\frac{Y_{DEP} - Y_{ARR}}{INT}$$ soit un nombre entier).

L'étape 1 d'étalonnage de position des deux ensembles infrarouges 4, 6 et 8, 10 comprend ensuite les opérations automatisées suivantes :

a) déplacement du socle 26 suivant la direction Z jusqu'à la position $Z_{mesure}$, par actionnement du vérin 78,
b) déplacement angulaire du plateau tournant 70 afin de transporter l'objet étalon en dimension edim jusqu'à sa position initiale de mesure par rapport à l'unité 2,
c) déplacement du premier ensemble infrarouge 4, 6 suivant la direction Y jusqu'à sa position de départ Y(1), par actionnement du vérin 76,

d) déplacement progressif, par incréments successifs de INT, du premier ensemble infrarouge 4, 6 suivant la direction Y, en l'éloignant du deuxième ensemble infrarouge 8, 10 fixé à une position $Y_{FIX}$, entre les positions Y(1) et Y(N), et détermination simultanée de la réponse infrarouge RI(n) de l'objet edim, correspondant à chaque position Y(n), de la manière suivante :

d-1) déplacement angulaire du plateau tournant 70 afin de transporter l'objet étalon en dimension edim à sa position finale de mesure,

d-2) mesure de la réponse infrarouge RI(n) dudit objet étalon en dimension edim,

d-3) déplacement angulaire du plateau tournant 70 afin de ramener l'objet étalon en dimension edim à sa position initiale de mesure,

e) calcul de la réponse infrarouge optimale $\quad RI_{OPT} = \dfrac{RI_{MAX} - RI_{MIN}}{2}$

ou : $RI_{MIN}$ est la valeur de la saturation minimale de la réponse infrarouge: au début de l'étalonnage, l'écartement des deux ensembles infrarouges 4, 6, et 8, 10 est très inférieur à la dimension significative $x_{edim}$ de l'objet étalon en dimension edim ; par suite, lorsque 50% du premier faisceau infrarouge est intercepté par l'objet edim, 100% du deuxième faisceaux infrarouge est intercepté par cet objet edim ; les premières réponses infrarouges ont alors une valeur identique $RI_{MIN}$ dite « saturée »,

et : $RI_{MAX}$ est la valeur de la saturation maximale de la réponse infrarouge : à la fin de l'étalonnage, l'écartement des deux ensembles infrarouges 4, 6, et 8, 10 est très supérieur à la dimension significative $x_{edim}$ de l'objet étalon en dimension edim ; par sui te, lorsque 50% du premier faisceau infrarouge est intercepté par l'objet edim, 0% du deuxième faisceau infrarouge est intercepté par cet objet edim ; les dernières réponses infrarouges ont alors une valeur identique $RI_{MAX}$ dite « saturée »,

f) calcul de la position optimale $Y_{OPT}$ du premier ensemble infrarouge 4, 6 par rapport au deuxième ensemble infrarouge 8, 10 : la réponse infrarouge optimale $RI_{OPT}$ est comprise entre deux valeurs successives RI(j) et RI(k) précédemment calculées de la réponse infrarouge, qui correspondent respectivement à deux positions Y(j) et Y(k) du premier ensemble infrarouge 4, 6 ; la position optimale YOPT s'en déduit de la manière suivante :

$$\text{si} \quad \frac{RI_{OPT} - RI(j)}{RI_{OPT} - RI(k)} < 1 \quad , \text{ alors } Y_{OPT} = Y(j)$$

$$\text{si} \quad \frac{RI_{OPT} - RI(j)}{RI_{OPT} - RI(k)} > 1 \quad , \text{ alors } Y_{OPT} = Y(k)$$

Les opérations a) à f) ci-dessus peuvent être renouvelées avec autant d'autres objets étalons en dimension edim que nécessaire.

A l'issue de l'étape 1 d'étalonnage de position des deux ensembles infrarouges 4, 6 et 8, 10, il est créé un premier fichier d'étalonnage qui comporte, notamment, l'écartement optimal d des deux ensembles infrarouges 4, 6 et 8, 10 correspondant sensiblement à la dimension significative des objets $d = ||Y_{FIX} - Y_{OPT}||$.

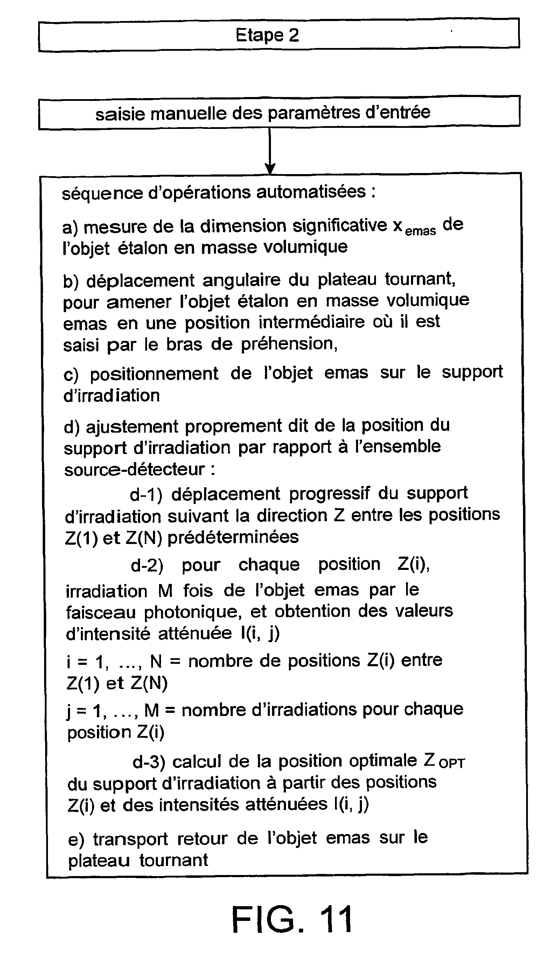

L'étape 2 d'étalonnage de position du support d'irradiation 90 de l'unité 30 de détermination par spectrométrie gamma de l'intensité du faisceau photonique atténuée par la traversée des objets 100 est illustrée de façon synthétique sur la figure 11.

Cette étape 2 d'étalonnage consiste à ajuster la position suivant la direction Z du support d'irradiation 90 par rapport à la source 32 et au détecteur 40 associé, afin de fixer la position $Z_{OPT}$ suivant la direction Z de la face supérieure 92 du support d'irradiation 90 sur lequel sont positionnés les objets 100 traversés par le faisceau photonique, en fonction de la masse volumique p connue précisément d'un ou plusieurs objet(s) étalon(s) en masse volumique emas. En pratique, la position $Z_{OPT}$ est établie en déplaçant progressivement le support d'irradiation 90 suivant la direction Z, et en irradiant plusieurs fois l'objet étalon en masse volumique emas installé sur le support d'irradiation 90 pour chacune des positions de celui-ci. Elle résulte du calcul d'un minimum d'une régression polynomiale d'ordre 4. Elle comporte une étape de détermination de la dimension significative $x_{emas}$ de chaque objet emas étalon en masse volumique.

L'étape 2 d'étalonnage de position du support d'irradiation 90 de l'unité 30 comprend tout d'abord une saisie par l'opérateur d'un ensemble de paramètres d'entrée au moyen d'un module interactif. Ces paramètres comprennent :

- la configuration des composants qui ont un déplacement micrométrique : vérins 96, 98, ce qui permet de gérer leur cinématique : position, vitesse, accélération,
- la configuration du plateau tournant 70, c'est-à-dire la nature des objets qui occupent les différents emplacements sur le plateau tournant 70 : objet quelconque 100, ou objet étalon en dimension edim, ou objet étalon en masse volumique emas, ou emplacement libre,
- l'emplacement occupé par les objets étalons en masse volumique emas sur le plateau tournant 70, cet emplacement étant un nombre allant de 1 à 12 pour l'exemple illustré,
- la durée de mesure ou temps de comptage,
- les positions Z(1) et Z(N) bornant l'intervalle de déplacement du support d'irradiation 90 suivant la direction Z,
- le nombre M de mesures de l'intensité photonique atténuée par la traversée de l'objet, pour chaque position Z(i) occupée par le support d'irradiation, pour i = 1, ..., N.

L'étape 2 d'étalonnage de position du support d'irradiation 90 de l'unité 30 comprend ensuite les opérations automatisées suivantes :

a) détermination de la dimension significative $x_{emas}$ de l'objet étalon en masse volumique, conformément à l'étape 4 qui sera décrite ci-après,
b) déplacement angulaire du plateau tournant 70, d'un angle A, afin de transporter l'objet étalon en masse volumique emas en une position intermédiaire où il est saisi par le bras de préhension 80,
c) positionnement de l'objet emas sur le support d'irradiation 90, qui comporte les sous-opérations suivantes :

c-1) déplacement du support d'irradiation 90, suivant la direction Z et vers le bas, par actionnement du vérin 98,
c-2) déplacement du bras de manutention 80 depuis sa position d'attente jusqu'à la verticale de la position intermédiaire de l'objet emas, par actionnement du vérin 86,
c-3) saisie, par le bras de manutention 80, de l'objet emas, et transport de celui-ci jusqu'à la verticale de la face supérieure 92 du support d'irradiation 90, par actionnement du vérin 86,
c-4) déplacement du support d'irradiation 90 jusqu'à la position Z(1), suivant la direction Z et vers le haut, par actionnement du vérin 98,
c-5) dépose de l'objet emas sur la face supérieure 92 du support d'irradiation 90, par le bras de manutention 80, par actionnement du vérin 86,
c-6) déplacement retour du bras de manutention 80 jusqu'à sa position d'attente, par actionnement du vérin 86,
c-7) mise en butée de l'objet emas sur la face supérieure 92 suivant la direction Y, par exemple par une opération de soufflage, qui se déroule de la manière suivante :

- déplacement du support d'irradiation 90 suivant la direction Z et vers le bas jusqu'à une position dite de soufflage, dans laquelle l'objet se trouve en regard d'un dispositif de soufflage prévu dans le système,
- envoi d'air comprimé provenant du dispositif de soufflage sur l'objet emas, suivant la direction Y, afin de plaquer celui-ci contre une butée 93 du support d'irradiation 90,

d) ajustement proprement dit de la position du support d'irradiation 90 par rapport à la source 32 et au détecteur associé 40, qui comporte les sous-opérations suivantes :

d-1) déplacement progressif du support d'irradiation 90 suivant la direction Z entre la position Z(1) prédéterminée et la position Z(N) prédéterminée,
d-2) pour chaque position Z(i), i = 1, ..., N, irradiation de l'objet étalon en masse volumique emas par le faisceau photonique, un nombre M de fois, ce qui conduit à un ensemble de valeurs d'intensité atténuée I(i, j), où i = 1, ..., N représente le nombre de positions successives Z(i) occupées par le support d'irradiation 90 et j = 1, ..., M représente le nombre d'irradiations effectuées à chaque position. Z(i),
d-3) calcul de la position optimale $Z_{OPT}$ du support d'irradiation 90 à partir d'une régression polynomiale d'ordre 4 des positions Z(i) par rapport aux intensités atténuées I(i, j), cette régression polynomiale d'ordre 4 étant prédéterminée et intégrée comme une donnée de l'unité 200 d'acquisition, de traitement et d'analyse,

e) transport retour de l'objet étalon en masse volumique emas sur le plateau tournant 70, par une séquence d'opérations inverses aux sous-opérations c-1) à c-6) qui ont été détaillées ci-dessus.

A l'issue de l'étape 2 d'étalonnage de la position du support d'irradiation 90 de l'unité 30 de détermination par spectrométrie gamma de l'intensité du faisceau photonique atténuée par la traversée des objets 100 il est crée un deuxième fichier d'étalonnage qui comporte, notamment, la position optimale $Z_{OPT}$ du support d'irradiation 90 suivant la direction Z.

L'étape 3 d'étalonnage de mesure de l'unité 30 de détermination par spectrométrie gamme comprend les opérations automatisées suivantes :

a) détermination de l'intensité photonique $I_{emas}$ atténuée par la traversée d'un objet étalon en masse volumique emas pris comme référence,

b) calcul du coefficient massique d'atténuation $\mu_m$ de l'objet étalon en masse volumique, et par suite de tous les objets du lot d'objets par la relation :

$$\rho_{emas} = -\frac{1}{\mu_m x_{emas}} \cdot L_n \frac{I_{emas}}{I_0} \; .$$

A l'issue de l'étape 3 d'étalonnage de mesure de l'unité 30 de détermination par spectrométrie gamma de l'intensité du faisceau photonique atténuée par la traversée des objets 100, il est crée un troisième fichier d'étalonnage qui comporte, notamment, l'intensité photonique $I_{emas}$ atténuée par la traversée de l'objet étalon en masse volumique emas.

Les étapes de détermination proprement dites respectent elles aussi une chronologie pré-établie et concernent les opérations suivantes :

- étape 4 : détermination de la dimension significative x de l'objet 100 à contrôler,
- étape 5 : transport de l'objet 100 vers le support d'irradiation 90,
- étape 6 : ajustement de la position de l'objet 100 par ajustement de la position du support d'irradiation 90 par rapport à la source 32 et au détecteur associé 40,
- étape 7 : détermination de l'intensité atténuée I du faisceau photonique transmis à travers l'objet 100,
- étape 8 : acquisition, traitement et analyse du spectre obtenu,

- étape 9 : détermination de la variation relative $\dfrac{\Delta\rho}{\rho}$ de masse volumique de l'objet 100 par rapport à celle d'un ou plusieurs objet(s) étalon(s) en masse volumique emas,
- étape 10 : transport retour de l'objet 100 jusqu'à son emplacement sur le plateau tournant 70.

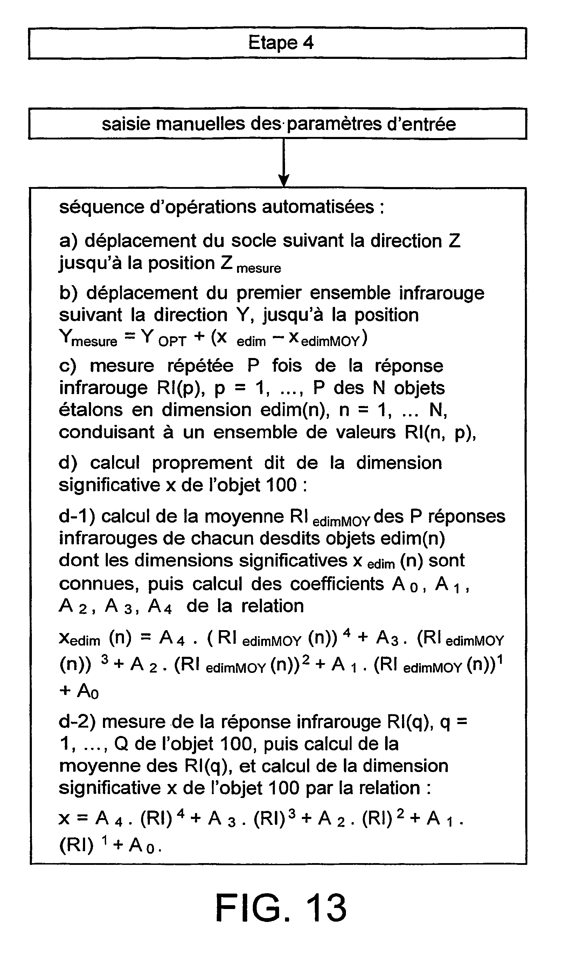

L'étape 4 de détermination de la dimension significative x de l'objet 100 à contrôler est illustrée de façon synthétique à la figure 13. Elle comprend tout d'abord une saisie par l'opérateur d'un ensemble de paramètres d'entrée au moyen d'un module interactif. Ces paramètres comprennent :

- la configuration des composants qui ont un déplacement micrométrique : vérins 76, 78, ce qui permet de gérer leur cinématique : position, vitesse, accélération,
- la configuration du plateau tournant 70, c'est-à-dire la nature des objets qui occupent les différents emplacements sur le plateau tournant 70 : objet quelconque 100, ou objet étalon en dimension edim, ou objet étalon en masse volumique emas, ou emplacement libre,
- l'emplacement occupé par l'objet 100 sur le plateau tournant 70, cet emplacement étant un nombre allant de 1 à 12 pour l'exemple illustré,
- la position $Z_{mesure}$ suivant la direction Z du socle 26 de l'unité 2, qui correspond à une cote z sur l'objet 100 par rapport à la base de celui-ci,
- le nombre P de mesures infrarouges pour chaque objet étalon en dimension edim(n), n = 1, ..., N, où N est le nombre d'objets étalons en dimension,
- le nombre Q de mesures infrarouges pour l'objet 100.

L'étape 4 de détermination de la dimension significative x de l'objet 100 à contrôleur utilise aussi les données contenues dans le premier fichier d'étalonnage issu de l'étape 1.

L'étape 4 de détermination de la dimension significative x de l'objet 100 à contrôler comprend ensuite les opérations automatisées suivantes :

a) déplacement du socle 26 suivant la direction Z jusqu'à la position $Z_{mesure}$, pzar actionnement du vérin 78,

b) déplacement du premier ensemble infrarouge 4, 6 suivant la direction Y, par actionnement du vérin 76, jusqu'à

une position $Y_{mesure}$ définie par :

$$Y_{mesure} = Y_{OPT} + (x_{edim} - x_{edimMOY})$$

où :

$Y_{OPT}$ est la position optimale provenant de l'étape 1 d'étalonnage, cette valeur étant contenue dans le premier fichier d'étalonnage,

$x_{edim}$ est la dimension de l'objet étalon en dimension edim utilisé lors de l'étape 1 d'étalonnage, cette valeur étant contenue dans le premier fichier d'étalonnage,

$x_{edimMOY}$ est la dimension significative moyenne de tous les objets edim étalons en dimension, cette valeur étant fournie par le fabricant,

c) mesure répétée P fois de la réponse infrarouge RI(p), p = 1, ..., P des N objets étalons en dimension edim(n), n = 1, ... N, ce qui conduit à un ensemble de valeurs RI(n, p),

d) calcul proprement dit de la dimension significative x de l'objet 100 de la manière suivante :

d-1) calcul de la moyenne $RI_{edimMOY} = \dfrac{\sum RI(n, p)}{P}$ des réponses infrarouges de chaque objet étalon en dimension edim(n) dont la dimension significative $x_{edim}$ (n) est connue, et mise en oeuvre d'une régression polynomiale d'ordre 4 des dimensions significatives $x_{edim}$ (n) pour calculer les coefficients $A_0$, $A_1$, $A_2$, $A_3$, $A_4$ d'une relation du type :

$$x_{edim}(n) = A_4 \cdot (RI_{edimMOY} (n))^4 + A_3 \cdot (RI_{edimMOY} (n))^3 +$$
$$A_2 \cdot (RI_{edimMOY} (n))^2 + A_1 \cdot (RI_{edimMOY} (n))^1 + A_0,$$

d-2) mesure, répétée Q fois, de la réponse infrarouge. RI(q), q = 1, ..., Q de l'objet à contrôler 100, calcul de la

moyenne $RI = \dfrac{\sum RI(q)}{Q}$ de ces réponses infrarouges, et calcul de la dimension significative x recherchée de l'objet 100 par la relation :

$$x = A_4 \cdot (RI)^4 + A_3 \cdot (RI)^3 + A_2 \cdot (RI)^2 + A_1 \cdot (RI)^1 + A_0$$

L'étape 5 de transport de l'objet 100 à contrôler vers le support d'irradiation est une étape automatisée qui reprend la séquence des sous-opérations b) et c) de l'étape 2 d'étalonnage qui ont été détaillées ci-dessus.

L'étape 6 d'ajustement de la position de l'objet 100 par rapport à la source 32 et au détecteur associé 40 est une étape automatisée qui reprend la sous-opération d) de l'étape 2 d'étalonnage qui a été détaillée ci-dessus.

L'étape 7 de détermination de l'intensité photonique I du faisceau photonique, atténuée par la traversée de l'objet 100 consiste en une mesure d'activité, qui est ensuite acquise, traitée et interprétée de manière connue en soi.

L'étape 8 d'acquisition, de traitement et d'analyse du spectre obtenu est une étape automatisée qui met en oeuvre des algorithmes de calculs connus en soi qui sont exécutés par le logiciel dédié impanté sur l'ordinateur 170 de l'unité 200 d'acquisition, de traitement et d'analyse.

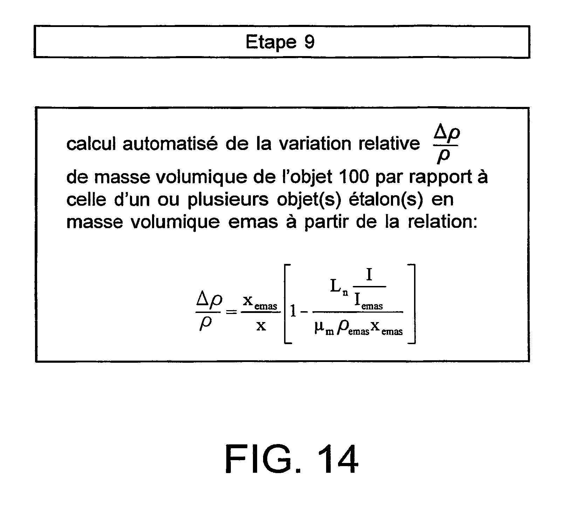

L'étape 9 de détermination de la variation relative $\dfrac{\Delta\rho}{\rho}$ de masse volumique de l'objet 100 par rapport à celle d'un ou plusieurs objet(s) étalon(s) en masse volumique emas est illustrée de façon synthétique à la figure 14. C'est une étape automatisée de calcul dans laquelle interviennent l'équation $\dfrac{\Delta\rho}{\rho} \approx \dfrac{x_{emas}}{x}\left[1-\dfrac{L_n\dfrac{I}{I_{emas}}}{\mu_m\rho_{emas}x_{emas}}\right]$ et les données qui ont été déterminées aux différentes étapes précédentes.

L'étape 10 de transport retour de l'objet 100 sur son emplacement du plateau tournant 70 est une étape automatisée qui reprend la sous-opération e) de l'étape 2 d'étalonnage qui a été détaillée ci-dessus.

Le procédé qui vient d'être décrit est mis en oeuvre au moyen d'un logiciel dédié. Ce logiciel comporte cinq modules indépendants, et un menu principal interactif, au moyen duquel un opérateur choisit de faire exécuter l'un des cinq modules. Les cinq modules regroupent les fonctions suivantes :

- premier module : détermination de la masse volumique d'un objet, qui recouvre l'étape 3 d'étalonnage et les étapes 4 à 10 de détermination proprement dite de la masse volumique,
- deuxième module : détermination de la dimension significative d'un objet,
- troisième module : étalonnage de position de l'unité de détermination de dimension significative,
- quatrième module : étalonnage de positions de l'unité de détermination de l'intensité photonique atténuée,
- cinquième module : gestion des fichiers de données.

EXEMPLE

Le système et le procédé décrits précédemment ont été testés.

La source était une source de $^{133}$Ba de 10 mCi d'activité. La durée des acquisitions était de l'ordre de 20 minutes.

Les mesures ont été effectuées sur un lot de 7 pastilles d'oxyde d'uranium ($UO_2$) dont les caractéristiques : diamètre, hauteur, masse volumique ou densité, apparaissent dans le tableau I :

| N⁰ pastille : i | 1 | 2 | 3 (étalon) |
|---|---|---|---|
| Diamètre (mm) | 8,165 | 8,143 | 8,166 |
| Hauteur (mm) | 11,54 | 11,44 | 11,27 |
| Masse volumique $(g.cm^3)$ | 10,260 ± 0.003 | 10,130 ± 0.003 | 9,900 ± 0.003 |
| écart-type $(g.cm^3)$ | $1,99.10^{-2}$ | $1,98.10^{-2}$ | $1,96.10^{-2}$ |

Tableau I

| N⁰ pastille : i | 4 | 5 | 6 | 7 |
|---|---|---|---|---|
| Diamètre (mm) | 8,147 | 8,123 | 8,117 | 8,169 |
| Hauteur (mm) | 11,49 | 11,29 | 11,54 | 11,59 |
| Masse volumique $(g.cm^3)$ | 10,150 ± 0.003 | 9,950 ± 0.003 | 9,960 ± 0.003 | 10,070 ± 0.003 |
| écart-type $(g.cm^3)$ | $1,98.10^{-2}$ | $1,95.10^{-2}$ | $1,93.10^{-2}$ | $1,97.10^{-2}$ |

Tableau I (suite)

La pastille 3 est prise comme pastille étalon.

L'objectif des mesures est la détermination précise, au moyen du système et du procédé de l'invention, de la variation relative de masse volumique des pastilles (1,2,4,5,6 et 7) par rapport à la masse volumique de la pastille étalon (pastille 3). La relation suivante s'applique :

$$\frac{\Delta\rho}{\rho} = \frac{x}{x_i}\left[1 - \frac{Ln\left(\frac{I_i}{I}\right)}{\mu_m\rho x}\right] - 1$$

Les diamètres des pastilles supposées «inconnues » sont obtenus par l'étape de détermination de la dimension significative, ici le diamètre des pastilles, par rayonnement infrarouge.

Les résultats des comptages obtenus par spectrométrie gamma, pour chacune des six pastilles sont exposés dans le tableau II. Ils ont été obtenus en suivant scrupuleusement la chronologie du procédé présentée précédemment.

Tableau II

| N° PASTILLE | I (en coups) | ECARTS DE MASSE VOLUMIQUE |
|---|---|---|
| 1 | $974725 \pm 1974$ | $(3,448633 \pm 0,017045).10^{-2}$ |
| 2 | $1012550 \pm 2012$ | $(2, 286541 \pm 0,061460).10^{-2}$ |
| 4 | $1009661 \pm 2010$ | $(2, 344449 \pm 0,016572).10^{-2}$ |
| 5 | $106388\text{-}E; \pm 2063$ | $(6, 611105 \pm 0,132441).10^{-3}$ |
| 6 | $1067853 \pm 2067$ | $(5, 941459 \pm 0,122442).10^{-3}$ |
| 7 | $10144895 \pm 2015$ | $(1, 873675 \pm 0,017101).10^{-3}$ |

Les écarts types relatifs aux variations de masses volumiques mesurées ont été estimés par un calcul de propagation d'incertitudes. Le tableau III est un tableau comparatif de ces résultats avec les écarts théoriques fournis par le fabriquant de pastilles.

| N° Pastille | 1 | 2 | 4 |
|---|---|---|---|
| $[\Delta\rho/\rho]_{théorique}$ | $3,63636.10^{-2}$ $\pm 2,77.10^{-3}$ | $2,32323.10^{-2}$ $\pm 2,78.10^{-3}$ | $2,52525.10^{-2}$ $\pm 2,77.10^{-3}$ |
| $[\Delta\rho/\rho]_{mesuré}$ | $3,44863.10^{-2}$ $\pm 1,70.10^{-4}$ | $2,28654.10^{-2}$ $\pm 1,65.10^{-4}$ | $2,34445.10^{-2}$ $\pm 1,66.10^{-4}$ |

Tableau III

| N° Pastille | 5 | 6 | 7 |
|---|---|---|---|
| $[\Delta\rho/\rho]théorique$ | $5,0505.10^{-3}$ $\pm 2,79.10^{-4}$ | $6,0606.10^{-3}$ $\pm 2,77.10^{-4}$ | $1,71717.10^{-2}$ $\pm 2,76.10^{-3}$ |
| $[\Delta\rho/\rho]mesuré$ | $6,611.10^{-3}$ $\pm 1,32.10^{-4}$ | $5,9415.10^{-3}$ $\pm 1,22.10^{-4}$ | $1,8737.10^{-2}$ $\pm 1,71.10^{-4}$ |

Tableau III (suite)

Ces résultats sont illustrés par le graphique de la figure 15. Les cercles représentent les valeurs de $\dfrac{\Delta\rho}{\rho}$ résultants de la mesure, alors que les croix représentent les valeurs de $\dfrac{\Delta\rho}{\rho}$ fournis par le fabricant. L'intervalle matérialisé représente l'écart-type calculé à partir de données fournies par le fabricant.

Ces résultats montrent que le système et le procédé selon l'invention permettent de détecter une variation relative de masse volumique d'environ $6.10^{-3}$ par rapport à la pastille choisie comme objet étalon.

**Revendications**

1.  Système, pour la détermination automatique de la masse volumique d'un objet (100) appartenant à un lot d'objets, **caractérisé en ce qu'**il comprend :

    - une unité (2) de détermination d'une dimension significative (x) dudit objet (100),
    - une unité (30) de détermination de l'intensité (I) d'un faisceau photonique, atténuée par la traversée dudit objet (100),
    - une unité d'acquisition, de traitement et d'analyse (200),
    - des moyens de transport (70, 72, 80, 82, 84, 86, 88) de l'objet (100) vers l'unité (2) de détermination de sa dimension significative (x) et vers l'unité (30) de détermination de l'intensité photonique atténuée,
    - des premiers moyens d'ajustement (74, 76, 78) de la position de l'objet (100) relativement à l'unité de détermination de sa dimension significative (x), et
    - des deuxièmes moyens d'ajustement (90, 92, 94, 96, 98) de la position de l'objet (100) relativement à l'unité (30) de détermination de l'intensité photonique atténuée,

    **en ce que** lesdits premiers moyens d'ajustement et deuxièmes moyens d'ajustement sont capables de déplacer l'objet (100) avec une précision de l'ordre du micron par rapport à une plaque de support (150) sur laquelle sont installés les éléments constitutifs du système,
    et **en ce que** la position de l'objet (100) relativement à l'unité (30) de détermination de l'intensité atténuée (I) est ajustée en fonction de la dimension significative (x) dudit objet (100).

2.  Système selon la revendication 1, **caractérisé en ce que** l'unité (200) d'acquisition, de traitement et d'analyse comprend un ordinateur (170) dans lequel est implanté un logiciel dédié qui exécute des séries d'instructions et des algorithmes de calcul automatique de la masse volumique de l'objet (100) .

3.  Système selon la revendication 1 ou 2, **caractérisé en ce que** l'unité (200) d'acquisition, de traitement et d'analyse fournit la variation relative $\left( \dfrac{\Delta\rho}{\rho} \right)$ de la masse volumique (p) de l'objet (100) par rapport à celle, connue, d'au moins un objet étalon (emas) appartenant au même lot d'objets (100).

4.  Système selon l'une quelconque des revendications 1 à 3, **caractérisé en ce que** l'unité (2) de détermination de la dimension significative de l'objet (100) comprend :

    - un premier ensemble infrarouge (4, 6) constitué d'un premier émetteur infrarouge (4) et d'un premier récepteur infrarouge (6),
    - un deuxième ensemble (8, 10) constitué d'un deuxième émetteur infrarouge (8) et d'un deuxième récepteur infrarouge (10),

    les deux ensembles infrarouges (4, 6 ; 8, 10) étant éloignés l'un de l'autre d'une distance connue (d), et émettant des faisceaux infrarouges qui sont parallèles entre eux,
    et la dimension significative (x) de l'objet (100) étant déduite de la réponse infrarouge obtenue lorsque l'objet (100) est déplacé de manière à intercepter successivement le premier faisceau et le deuxième faisceau infrarouges, selon une direction sensiblement perpendiculaire à celle des axes (12, 14) des deux faisceaux, ladite réponse infrarouge correspondant à la fraction (24) du deuxième faisceau non encore interceptée par l'objet (100) lorsqu'il intercepte encore la moitié (22) du premier faisceau.

5.  Système selon la revendication 4, **caractérisé en ce que** l'unité (2) de détermination comprend en outre un troisième ensemble (16, 18) émetteur-récepteur photoélectrique, disposé en amont du premier ensemble infrarouge (4, 6) par rapport au deuxième ensemble infrarouge (8, 10), et destiné à l'ajustement préalable de l'intensité des deux faisceaux infrarouges.

6.  Système selon la revendication 4 ou 5, **caractérisé en ce que** la dimension significative (x) de l'objet (100) est obtenue après avoir fait déplacer ledit objet QN fois et mesuré Q réponses infrarouges RI(q), pour q compris entre 1 et Q, par une relation du type :

$$x = A_4 \cdot (\text{moyenne RI}(q))^4 + A_3 \cdot (\text{moyenne RI}(q))^3 +$$
$$A_2 \cdot (\text{moyenne RI}(q))^2 + A_1 \cdot (\text{moyenne RI}(q))^1 + A_0 ,$$

où :

$A_0$, $A_1$, $A_2$, $A_3$, $A_4$ sont des coefficients obtenus précédemment en appliquant la même relation à au moins quatre objets étalons en dimension (edim), pour lesquels une réponse infrarouge RI(edim) est mesurée.

7. Système selon l'une quelconque des revendications 1 à 6, **caractérisé en ce que** l'unité (30) de détermination de l'intensité atténuée d' un faisceau photonique est une unité de détermination par spectrométrie gamma, qui comprend :

- un ensemble (32) formé d'une source et d'un collimateur,
- un ensemble (40) formé d'un détecteur et d'un collimateur,
- une chaîne d'acquisition et de comptage des photons gamma (48).

8. Système selon la revendication 7, **caractérisé en ce que** la chaîne d'acquisition et de comptage (48) comprend :

- un détecteur à germanium de haute densité,
- un préamplificateur (50),
- un module DSP (Digital Signal Processor) (52),
- un module haute tension (54),
- un module réseau (56),
- un ordinateur d'acquisition de données (170),
- un cryostat (60).

9. Système selon l'une quelconque des revendications 1 à 8, **caractérisé en ce que** les moyens de transport (70, 72, 80, 82, 84, 86, 88) comprennent un plateau tournant (70) et un moteur pas à pas (72) d'entraînement dudit plateau (70).

10. Système selon l'une quelconque des revendications 1 à 9, **caractérisé en ce que** les moyens de transport comprennent un bras de manutention (80).

11. Système selon la revendication 10, **caractérisé en ce que** le bras de manutention (80) est un bras articulé équipé d'une pince d'extrémité (82) destinée à saisir et déposer l'objet (100).

12. Système selon l'une quelconque des revendications 3 à 11, **caractérisé en ce que** les premiers moyens d'ajustement comprennent :

- une glissière (74) pour fixer la position d'un socle (26) de l'unité (2) de détermination de la dimension significative de l'objet selon une direction X.
- un vérin (76) pour déplacer le premier ensemble infrarouge (4, 6) par rapport le deuxième ensemble infrarouge (8, 10) de ladite unité (2) selon une direction Y perpendiculaire à la direction Y.
- un vérin (78) pour déplacer ledit socle (26) de ladite unité (2) selon une direction Z perpendiculaire au plan (X, Y).

13. Système selon l'une quelconque des revendications 1 à 12, **caractérisé en ce que** les deuxièmes moyens d'ajustement comprennent un support d'irradiation (90) sur lequel est installé l'objet (100) entre une source (32) et un détecteur (40) de l'unité (30) de détermination de l'intensité atténuée du faisceau photonique traversant l'objet (100).

14. Système selon la revendication 13, **caractérisé en ce que** les deuxièmes moyens d'ajustement comprennent :

- une glissière (94) pour fixer un support d'irradiation (90) selon une direction X,
- un vérin (96) pour déplacer, selon une direction Y perpendiculaire à la direction X, ledit support d'irradiation (90) entre une source (32) et un détecteur (40) de l'unité (30) de détermination de l'intensité atténuée du faisceau photonique traversant l'objet (100),
- un vérin (98) pour déplacer, selon une direction Z perpendiculaire au plan (X, Y), ledit support d'irradiation (90) entre une source (32) et un détecteur (40) de l'unité (30) de détermination de l'intensité atténuée du faisceau photonique traversant l'objet (100).

**15.** Procédé de mis e en oeuvre du système pour la détermination automatique de la masse volumique d'un objet (100) appartenant à un lot d'objets selon l'une quelconque des revendications 1 à 14, ledit système comportant une unité (2) de détermination d'une dimension significative (x) d'un objet (100) et une unité (300) de détermination de l'intensité (I) d'un faisceau photonique atténué par la traversée dudit objet (100), **caractérisé en ce qu'**il comprend les étapes d'étalonnage suivantes :

- une étape 1 d'étalonnage de position de deux ensembles infrarouges (4, 6 ; 8, 10) de l'unité (2) de détermination de la dimension significative des objets (100),
- une étape 2 d'étalonnage de position d'un support d'irradiation (90) de l'unité (30) de détermination par spectrométrie gamma de l'intensité du faisceau photonique atténuée par la traversée des objets (100),
- une étape 3 d'étalonnage de mesure de l'ensemble source-détecteur (32, 40) de l'unité (30), et **en ce qu'**il comprend des étapes de détermination proprement dite de la dimension significative (x) des objets (100), qui sont effectuées sur chaque objet (100) dudit lot d'objets.

**16.** Procédé selon la revendication 15, **caractérisé en ce que** l'étape 1 d'étalonnage comporte une saisie par l'opérateur d'un ensemble de paramètres d'entrée au moyen d'un module interactif, parmi lesquels :

- la configuration de composants qui ont un déplacement micrométrique, parmi les quels deux vérins (76, 78),
- la configuration du plateau tournant (70), c'est-à-dire la nature des objets qui occupent différents emplacements prévus sur celui-ci,
- l'emplacement occupé par chaque objet étalon en dimension (edim) sur le plateau tournant (70),
- une position ($Z_{mesure}$) d'un socle (26) de l'unité (2) suivant la direction Z,
- des positions (Y(l), Y(N)) bornant un intervalle de déplacement du premier ensemble infrarouge (4, 6) suivant la direction Y,
- un pas (INT) de déplacement du premier ensemble infrarouge (4, 6) suivant la direction Y.

**17.** Procédé selon la revendication 16, **caractérisé en ce que** l'étape 1 d'étalonnage comporte en outre les opérations suivantes :

a) déplacement du socle (26) suivant la direction Z jusqu'à la position ($Z_{mesure}$),
b) déplacement angulaire du plateau tournant (70) afin de transporter un objet étalon en dimension (edim) jusqu'à sa position initiale de mesure par rapport à l'unité (2),
c) déplacement du premier ensemble infrarouge (4, 6) suivant la direction Y jusqu'à sa position de départ (Y(1)),
d) déplacement progressif, par incréments successifs de (INT), du premier ensemble infrarouge (4, 6) suivant la direction Y, en l'éloignant du deuxième ensemble infrarouge (8, 10) fixé à une position $Y_{FIX}$, entre les positions Y(1) et Y(N), et détermination simultanée de la réponse infrarouge (RI(n)) de l'objet (edim), correspondant à chaque position (Y(n))
e) calcul de la réponse infrarouge optimale $RI_{OPT}$,
f) calcul de la position optimale $Y_{OPT}$ du premier ensemble infrarouge (4, 6) par rapport au deuxième ensemble infrarouge (8, 10).

**18.** Procédé selon la revendication 17, **caractérisé en ce que** l'opération d) de déplacement progressif comporte les sous-opérations suivantes :

d-1) déplacement angulaire du plateau tournant (70) afin de transporter l'objet étalon en dimension (edim) à sa position finale de mesure,
d-2) mesure de la réponse infrarouge (RI(n)) dudit objet étalon en dimension (edim),
d-3) déplacement angulaire du plateau tournant (70) afin de ramener l'objet étalon en dimension (edim) à sa position initiale de mesure.

**19.** Procédé selon la revendication 17 ou 18, **caractérisé en ce que** la réponse infrarouge optimale $RI_{OPT}$ est obtenue par la relation :

$$RI_{OPT} = \frac{RI_{MAX} - RI_{MIN}}{2}$$

où : $RI_{MIN}$ est la valeur de la saturation minimale de la réponse infrarouge ,
et : $RI_{MAX}$ est la valeur de la saturation maximale de la réponse infrarouge.

**20.** Procédé selon l'une quelconque des revendications 17 à 19, **caractérisé en ce que** l'opération f) de calcul de la position optimale $Y_{OPT}$ est obtenue de la manière suivants :

$$\text{si } \frac{RI_{OPT} - RI(j)}{RI_{OPT} - RI(k)} < 1 \text{ , alors } Y_{OPT} = Y(j)$$

$$\text{si } \frac{RI_{OPT} - RI(j)}{RI_{OPT} - RI(k)} > 1 \text{ , alors } Y_{OPT} = Y(k)$$

où RI(j) et RI(k) sont deux valeurs précédemment calculées de la réponse infrarouge entre lesquelles est comprise la réponse optimale recherchée $RI_{OPT}$, qui correspondent respectivement à deux positions Y(j) et Y(k) du premier ensemble infrarouge (4, 6).

**21.** Procédé selon l'une quelconque des revendications 15 à 20, **caractérisé en ce que** l'étape 2 d'étalonnage comporte une saisie par l'opérateur d'un ensemble de paramètres d'entrée au moyen d'un module interactif, parmi lesquels :

- la configuration de composants qui ont un déplacement micrométrique, parmi lesquels deux vérins (96, 98),
- la configuration du plateau tournant (70), c'est-à-dire la nature des objets qui occupent différents emplacements sur celui-ci,
- l'emplacement occupé par chaque objet étalon en masse volumique (emas) sur le plateau tournant (70),
- une durée de mesure ou temps de comptage,
- des positions Z(1) et Z(N) bornant un intervalle de déplacement d'un support d'irradiation (90) suivant la direction Z,
- un nombre M de mesures de l'intensité photonique atténuée par la traversée de chaque objet étalon en masse volumique, pour chaque position Z(i) occupée par le support d'irradiation, pour i = 1,...,N.

**22.** Procédé selon la revendication 21, **caractérisé en ce que** l'étape 2 d'étalonnage comporte en outre les opérations suivantes :

a) détermination de la dimension significative ($x_{mas}$) de chaque objet étalon en masse volumique (emas),
b) déplacement angulaire du plateau tournant (70), d'un angle (A), afin de transporter ledit objet étalon en masse volumique (emas) en une position intermédiaire où il est saisi par un bras de préhension (80),
c) positionnement dudit objet (emas) sur un support d'irradiation (90),
d) ajustement proprement dit de la position du support d'irradiation (90) par rapport à une source (32) et un détecteur associé (40),
e) transport retour de l'objet étalon en masse volumique (emas) sur le plateau tournant (70), par une opération inverse à l'opération c).

**23.** Procédé selon la revendications 22, **caractérisé en ce que** l'opération c) de positionnement de l'objet (emas) sur le support d'irradiation (90) comporte les sous-opérations suivantes :

c-1) déplacement du support d'irradiation (90), suivant la direction Z et vers le bas,
c-2) déplacement du bras de préhension (80) depuis sa position d'attente jusqu'à la verticale de la position intermédiaire de l'objet (emas),
c-3) saisie, par le bras de préhension (80), de l'objet emas, et transport de celui-ci jusqu'à la verticale d'une face supérieure (92) du support d'irradiation (90),

c-4) déplacement du support d'irradiation 90 jusqu'à la position Z(1), suivant la direction Z et vers le haut,
c-5) dépose de l'objet (emas) sur la face supérieure (92) du support d'irradiation (90), par le bras de manutention 80,
c-6) déplacement retour du bras de manutention (80) jusqu'à sa position d'attente,
c-7) mise en butée, suivant la direction Y, de l'objet (emas) sur la face supérieure (92).

**24.** Procédé selon la revendications 22 ou 23, **caractérisé en ce que** l'opération d) d'ajustement proprement dit de la position du support d'irradiation (90) par rapport à la source (32) et au détecteur associé (40) comporte les sous-opérations suivantes :

d-1) déplacement progressif du support d'irradiation (90) suivant la direction Z entre deux positions Z(1) et Z(N) prédéterminées,
d-2) pour chaque position Z(i), i = 1, ..., N, irradiation de l'objet étalon en masse volumique (emas) par le faisceau photonique, un nombre M de fois, ce qui conduit à un ensemble de valeurs d'intensité atténuée I(i, j), où i = 1, ..., N représente le nombre de positions successives Z(i) occupées par le support d'irradiation (90) et j = 1, ..., M représente le nombre d'irradiations effectuées à chaque position Z(i),
d-3) calcul de la position optimale ($Z_{OPT}$) du support d'irradiation (90) à partir d'une régression polynomiale d'ordre 4 des positions Z(i) par rapport aux intensités atténuées I(i, j), cette régression polynomiale d'ordre 4 étant prédéterminée et intégrée comme une donnée d'une unité (200) d'acquisition, de traitement et d'analyse.

**25.** Procédé selon l'une quelconque des revendications 15 à 24, **caractérisé en ce que** l'étape 3 d'étalonnage de l'unité 30 de détermination par spectrométrie gamma comprend les opérations automatisées suivantes :

a) mesure de l'intensité photonique ($I_{emas}$) atténuée par la traversée d'un objet étalon en masse volumique (emas)

b) calcul du coefficient massique d'atténuation ($\mu$m) de l'objet étalon en masse volumique, par la relation :

$$\rho_{emas} = -\frac{1}{\mu_m x_{emas}} \cdot L_n \frac{I_{emas}}{I_0}$$

**26.** Procédé selon l'une quelconque des revendications 15 à 25, **caractérisé en ce que** les étapes de détermination proprement dite comprennent :

- une étape 4 de détermination de la dimension significative (x) de l'objet (100) à contrôler,
- une étape 5 de transport de l'objet (100) vers un support d'irradiation (90),
- une étape 6 d'ajustement de la position de l'objet (100) par ajustement de la position du support d'irradiation (90) par rapport à une source (32) et un détecteur associé (40),
- une étape 7 de détermination de l'intensité atténuée (I) du faisceau photonique transmis à travers l'objet (100),
- une étape 8 d'acquisition, traitement et analyse du spectre obtenu,
- une étape 9 de détermination de la variation relative $\frac{\Delta\rho}{\rho}$ de masse volumique (p) de l'objet (100) par rapport à celle d'un ou plusieurs objet(s) étalon(s) en masse volumique (emas),
- une étape 10 de transport retour de l'objet (100) jusqu'à son emplacement sur le plateau tournant (70).

**27.** Procédé selon la revendication 26, **caractérisé en ce que** l'étape 4 de détermination de la dimension significative (x) de l'objet (100) à contrôler comporte une saisie par l'opérateur d'un ensemble de paramètres d'entrée au moyen d'un module interactif, parmi lesquels :

- la configuration d'un plateau tournant (70), c'est-à-dire la nature des objets qui occupent différents emplacements sur celui-ci,
- l'emplacement occupé par l'objet (100) sur le plateau tournant (70),
- une position ($Z_{mesure}$) suivant la direction Z du socle (26) de l'unité (2),
- un nombre P de mesures infrarouges pour chaque objet étalon en dimension (edim(n)), n = 1, ..., N, où N est le nombre d'objets étalons en dimension,

- un nombre Q de mesures infrarouges pour l'objet (100).

28. Procédé selon la revendication 27, **caractérisé en ce que** l'étape 4 de détermination de la dimension significative (x) de l'objet (100) à contrôler comporte en outre les opérations suivantes :

a) déplacement d'un socle (26) de l'unité (2) suivant la direction Z jusqu'à la position ($z_{mesure}$),
b) déplacement d'un premier ensemble infrarouge (4, 6) suivant la direction Y, jusqu'à une position ($Y_{mesure}$) définie par : $Y_{mesure} = Y_{OPT} + (X_{edim} - X_{edimMOY})$, où :

$Y_{OPT}$ est la position optimale provenant de l'étape 1 d'étalonnage,
$X_{edim}$ est la dimension de l'objet étalon en dimension (edim) utilisé lors de l'étape 1 d'étalonnage,
$X_{edimMOY}$ est la dimension significative moyenne de tous les objets (edim) étalons en dimension,

c) mesure répétée P fois de la réponse infrarouge RI(p), p = 1, ..., P des N objets étalons en dimension (edim (n)), n = 1, ... N, ce qui conduit à un ensemble de valeurs RI(n, p),
d) calcul proprement dit de la dimension significative de l'objet (100).

29. Procédé selon la revendication 28, **caractérisé en ce que** l'opération d) de calcul proprement dit de la dimension significative (x) de l'objet (100) est effectuée de la manière suivante :

d-1) mise en oeuvre d'une régression polynomiale d'ordre 4 des dimensions significatives $X_{edim}(n)$ de chacun des N objets étalons en dimension edim(n), en fonction de la moyenne des réponses infrarouges

$$RI_{edimMOY} = \frac{\sum RI(n, p)}{P}$$ dudit objet étalon en dimension edim(n), pour calculer les coefficients $A_0$,

$A_1$, $A_2$, $A_3$, $A_4$ d'une relation du type :

$$x_{edim}(n) = A_4 \cdot (RI_{edimMOY}(n))^4 + A_3 \cdot (RI_{edimMOY}(n))^3 +$$
$$A_2 \cdot (RI_{edimMOY}(n))^2 + A_1 \cdot (RI_{edimMOY}(n))^1 + A_0,$$

d-2) mesure répétée Q fois de la réponse infrarouge RI(q), q = 1, ..., Q de l'objet à contrôler 100, calcul de la moyenne $$RI = \frac{\sum RI(q)}{Q}$$ de ces réponses infrarouges, et calcul de la dimension significative x de l'objet 100 par la relation :

$$x = A_4 \cdot (RI)^4 + \cdot A_3 \cdot (RI)^3 + A_2 \cdot (RI)^2 + A_1 \cdot (RI)^1 + A_0$$

30. Utilisation du système selon l'une quelconque des revendications 1 à 14 et du procédé selon l'une quelconque des revendications 15 à 29, pour le contrôle d'objets (100) en cours de fabrication.

31. Utilisation selon la revendication 30, dans laquelle les objets (100) sont des pastilles de combustible nucléaire.

**Claims**

1. System for automatic determination of the density of an object (100) belonging to a set of objects, **characterised in that** it comprises:

- an apparatus (2) to determine a significant dimension (x) of said object (100),
- an apparatus (30) to determine the intensity (I) of a photon beam attenuated by passing through said object (100),
- an acquisition, processing and analysis apparatus (200),
- means (70, 72, 80, 82, 84, 86, 88) of transporting the object (100) to the apparatus (2) for determining its significant dimension (x) and towards the apparatus (30) for determining the attenuated photon intensity,
- first means (74, 76, 78) of adjusting the position of the object (100) relative to the apparatus for determining its significant dimension (x), and
- second means (90, 92, 94, 96, 98) of adjusting the position of the object (100) relative to the apparatus (30) for determining the attenuated photon intensity,

and **in that** said first and second adjustment means are capable of moving the object (100) with a precision of the order of one micron with respect to a support plate (150) on which the elements making up the system are installed, and **in that** the position of the object (100) relative to the apparatus (30) for determining the attenuated intensity (I) is adjusted as a function of the significant dimension (x) of said object (100).

2. System set forth in claim 1, **characterised in that** the acquisition, processing and analysis apparatus (200) includes a computer (170) in which a dedicated software is installed that runs series of instructions and calculation algorithms used in the automatic method for determination of the density of the object (100).

3. System set forth in either claim 1 or 2, **characterised in that** the acquisition, processing and analysis apparatus (200) gives the relative variation $\left(\dfrac{\Delta\rho}{\rho}\right)$ of the density (p) of the object (100), relative to the known density of at least one standard density object (emas) belonging to the same set of objects (100).

4. System set forth in any one of claims 1 to 3, **characterised in that** the apparatus (2) for determination of the significant dimension of the object (100) includes:

- a first infrared assembly (4, 6) composed of a first infrared emitter (4) and a first infrared receiver (6),

a second infrared assembly (8, 10) composed of a second infrared emitter (8) and a second infrared receiver (10), the two infrared assemblies (4, 6: 8, 10) being separated from each other by a known distance (d), and emitting infrared beams that are parallel to each other, and the significant dimension (x) of the object (100) is deduced from the infrared response obtained when the object (100) is moved so as to intercept the first infrared beam and the second infrared beam in sequence, along a direction substantially perpendicular to the direction of the axes (12, 14) of the two beams, said infrared response corresponding to the fraction (24) of the second beam non yet intercepted by the object (100) when it is still intercepting half (22) of the first beam.

5. System set forth in claim 4, **characterised in that** the determination apparatus (2) also includes a third photoelectric transceiver assembly (16, 18) arranged on the input side of the first infrared assembly (4, 6) with respect to the second infrared assembly (8, 10) and intended to make a prior adjustment of the intensity of the two infrared beams.

6. System set forth in either claim 4 or 5, **characterised in that** the significant dimension (x) of the object (100) is obtained after moving said object QN times and measuring Q infrared responses RI(q), where q is between 1 and Q, with a relation of the following type:

$$x = A_4.(\text{average RI}(q))^4 + A_3.(\text{average RI}(q))^3 + A_2.(\text{average RI}(q))^2 + A_1.(\text{average RI}(q))^1 + A_0,$$

where $A_0$, $A_1$, $A_2$, $A_3$, $A_4$, are coefficients obtained previously applying the same relation to at least four objects with standard dimension (edim), for which an infrared response $RI_{(edim)}$ is measured.

7. System set forth in any one of claims 1 to 6, **characterised in that** the apparatus (30) for determining the attenuated intensity of a photon beam is a gamma spectrometry determination apparatus, comprising:

- an assembly (32) formed from a source and a collimator,
- an assembly (40) formed from a detector and a collimator,
- a gamma photon acquisition and counting system (48).

8. System set forth in claim 7, **characterised in that** the acquisition and counting system (48) comprises:

- a high-density germanium detector,
- a preamplifier (50),
- a Digital Signal Professor (DSP) (52),
- a high voltage module (54),
- a network module (56),
- a data acquisition computer (170),
- a cryostat (60).

9. System set forth in any one of claims 1 to 8, **characterised in that** the transport means (70, 72, 80, 82, 84, 86, 88) comprise a turntable (70) and a stepping motor (72) driving said turntable (70).

10. System set forth in any one of claims 1 to 9, **characterised in that** the transport means comprise a handling arm (80).

11. System set forth in claim 10, **characterised in that** the handling arm (80) is an articulated arm equipped with an end clamp (82) intended to grip and put the object (100) down.

12. System set forth in any one of claims 3 to 11, **characterised in that** the first adjustment means comprise:

- a slide (74) to fix the position of a base (26) of the apparatus (2) for determining the significant dimension of the object along a direction X;
- an actuator (76) to bring the first infrared assembly (4, 6) closer to or further from the second infrared assembly (8, 10) of said apparatus (2) along a Y direction perpendicular to the X direction;
- an actuator (78) to move said base (26) of said apparatus (2) along a direction Z perpendicular to the plane (X, Y).

13. System set forth in any one of claims 1 to 12, **characterised in that** the second adjustment means comprise an irradiation support (90) onto which the object (100) is installed between a source (32) and a detector (40) in the apparatus (30) for determining the attenuated intensity of the photon beam passing through the object (100).

14. System set forth in claim 13, **characterised in that** the second adjustment means comprise:

- a slide (94) to fix an irradiation support (90) along a direction X,
- an actuator (96) to move said irradiation support (90) between a source (32) and a detector (40) in the apparatus (30) for determining the attenuated intensity of the photon beam passing through the object (100), along a direction X.
- an actuator (98) to move said irradiation support (90) between a source (32) and a detector (40) in the apparatus (30) for determining the attenuated intensity of the photon beam passing through the object (100), along a direction Z perpendicular to the plane (X, Y).

15. Method for using the system for automatically determining the density of an object (100) belonging to a set of objects according to any one of claims 1 to 14, said system comprising an apparatus (2) to determine a significant dimension (x) of an object (100) and an apparatus (30) to determine the intensity (I) of a photon beam attenuated by passing through said object (100), **characterised in that** it includes the following calibration steps:

- a step 1 to calibrate the position of two infrared assemblies (4, 6; 8, 10) in the apparatus (2) to determine the significant dimension of objects (100),
- a step 2 to calibrate the position of an irradiation support (90) of the gamma spectrometry apparatus (30) used to determine the intensity of the photon beam attenuated by passing through objects (100),
- a step 3 to calibrate the measurement of the source-detector (32, 40) assembly of the apparatus (30),

and **in that** it includes steps to actually determine the significant dimension (x) of objects (100), that are done on each object (100) in said set of objects.

**16.** Method set forth in claim 15, **characterised in that** the calibration step 1 includes operator input of a set of input parameters using an interactive module. These parameters include:

- configuration of components that have a micrometric displacement including two actuators (76, 78),
- configuration of the turntable (70), in other words the nature of the objects that occupy different locations provided on the turntable,
- the position occupied by each standard dimension object (edim) on the turntable (70),
- a position ($Z_{measure}$) along the Z direction of a base (26) of the apparatus (2),
- positions Y(1) and Y(N) limiting a displacement interval of the first infrared assembly (4, 6) along the Y direction,
- a displacement step (INT) of the first infrared assembly (4, 6) along the Y direction.

**17.** Method set forth in claim 16, **characterised in that** the calibration step 1 also includes the following operations:

a) displacement of the base (26) along the Z direction as far as the ($Z_{measure}$) position,
b) angular displacement of the turntable (70) so as to transport a standard dimension object (edim) as far as its initial measurement position with respect to the apparatus (2),
c) displacement of the first infrared assembly (4, 6) along the Y direction as far as its start position (Y(1)),
d) progressive displacement of the first infrared assembly (4, 6) along the Y direction in successive increments of (INT), moving it away from the second infrared assembly (8, 10) fixed at a position $Y_{FIX}$ between the Y(1) and Y(N) positions, and simultaneous determination of the infrared response (RI(n)) of the object (edim) corresponding to each position (Y(n)):
e) calculate the optimum infrared response $RI_{OPT}$,
f) calculate the optimum position $Y_{OPT}$ of the first infrared assembly (4, 6) with respect to the second infrared assembly (8, 10).

**18.** Method set forth in claim 17, **characterised in that** the operation d) for progressive displacement includes the following sub-operations:

d-1) angular displacement of the turntable (70) so as to transport the standard dimension object (edim) to its final measurement position,
d-2) measure the infrared response (RI(n)) of said standard dimension object (edim),
d-3) angular displacement of the turntable (70) so as to bring the standard dimension object (edim) to its initial measurement position.

**19.** Method set forth in either claim 17 or 18, **characterised in that** the optimum infrared response $RI_{OPT}$ is obtained using the relation:

$$RI_{OPT} = \frac{RI_{MAX} - RI_{MIN}}{2}$$

where: $RI_{MIN}$ is the value of the minimum saturation of the infrared response,
and: $RI_{MAX}$ is the value of the maximum saturation of the infrared response.

**20.** Method set forth in any one of claims 17 to 19, **characterised in that** the operation f) to calculate the optimum position $Y_{OPT}$ is obtained as follows:

$$\text{If } \frac{RI_{OPT} - RI(j)}{RI_{OPT} - RI(k)} < 1, \text{ then } Y_{OPT} = Y(j)$$

$$\text{If } \frac{RI_{OPT} - RI(j)}{RI_{OPT} - RI(k)} > 1, \text{ then } Y_{OPT} = Y(k)$$

where RI(j) and RI(k) are two previously calculated values of the infrared response between which the required optimum response $RI_{OPT}$ lies, that correspond to two positions Y(j) and Y(k) of the first infrared assembly (4, 6) respectively.

21. Method set forth in any one of claims 15 to 20, **characterised in that** the calibration step 2 includes operator input of a set of input parameters using an interactive module. These parameters include:

- configuration of components that have a micrometric displacement, including two actuators (96, 98),
- configuration of the turntable (70), in other words the nature of the objects that occupy different locations provided on the turntable,
- the position occupied by each standard density object (emas) on the turntable (70),
- a measurement duration or count time,
- positions Z(1) and Z(N) limiting a displacement interval of an irradiation support (90) along the Z direction,
- a number M of measurements of the photon intensity attenuated by passing through each standard density object, for each position Z(i) occupied by the irradiation support, for i=1,...,N.

22. Method set forth in claim 21, **characterised in that** the calibration step 2 also includes the following operations:

a) determination of the significant dimension ($X_{emas}$) of each standard density object (emas),
b) angular displacement of the turntable (70) by an angle (A), in order to transport said standard density object (emas) into an intermediate position in which it will be gripped by a gripping arm (80),
c) positioning of said object (emas) on an irradiation support (90),
d) actual adjustment of the position of the irradiation support (90) with respect to a source (32) and an associated detector (40),
e) return transport of the object with standard density (emas) on the turntable (70) repeating the sequence described in operation c) but in the reverse order.

23. Method set forth in claim 22, **characterised in that** operation c) to position the object (emas) on the irradiation support (90) includes the following sub-operations:

c-1) displacement of the irradiation support (90) downwards, along the Z direction,
c-2) displacement of the gripping arm (80) from its waiting position to become vertically in line with the intermediate position of the object (emas),
c-3) gripping the object emas by the gripping arm (80), and then transport of the object until it is vertically in line with the top face (92) of the irradiation support (90),
c-4) displacement of the irradiation support 90 as far as the position Z(1), upwards and along the Z direction,
c-5) put the object (emas) down on the top face (92) of the irradiation support (90) using the handling arm 80,
c-6) displacement and return of the handling arm (80) as far as its waiting position,
c-7) force the object (emas) into contact with a stop on the top face (92) along the Y direction.

24. Method set forth in either claim 22 or 23, **characterised in that** the operation d) to actually adjust the position of the irradiation support (90) with respect to the source (32) and the associated detector (40) includes the following sub-operations:

d-1) progressive displacement of the irradiation support (90) along the Z direction between two predetermined positions Z(1) and Z(N),
d-2) for each position Z(i), i = 1, ..., N, irradiation of the standard density object (emas) by the photon beam a number M of times, which leads to a set of values of attenuated intensity I (i, J), where i = 1, ..., N represents the number of successive positions Z(i) occupied by the irradiation support (90) and j = 1, ..., M represents the number of irradiations made at each position Z(i),
d-3) calculate the optimum position ($Z_{OPT}$) of the irradiation support (90) starting from an order 4 polynomial regression of positions Z(i) with respect to the attenuated intensities I(i, j), this order 4 polynomial regression being predetermined and integrated as a data item of an acquisition, processing and analysis apparatus (200).

25. Method set forth in any one of claims 15 to 24, **characterised in that** the step 3 to calibrate the measurement of the gamma spectrometry determination apparatus 30 includes the following automated operations:

a) measurement of the photon intensity ($I_{emas}$) attenuated by passing through a standard density object (emas),
b) calculate the attenuation mass coefficient ($\mu$m) of the standard density object, using the following relation:

$$\rho_{emas} = \frac{1}{\mu_m \, xemas} . L_n \frac{I_{emas}}{I_O}$$

26. Method set forth in any one of claims 15 to 25, **characterised in that** the actual determination steps also include:

- a step 4 to determine the significant dimension (x) of the object (100) to be tested,
- a step 5 to transport the object (100) towards the irradiation support (90),
- a step 6 to adjust the position of the object (100) by adjusting the position of the irradiation support (90) with respect to a source (32) and an associated detector (40),
- a step 7 to determine the attenuated intensity (I) of the photon beam transmitted through the object (100),
- a step 8 for acquisition, processing and analysis of the spectrum obtained,

- a step 9 to determine the relative variation $\dfrac{\Delta\rho}{\rho}$ of the density ($\rho$) of the object (100), relative to the density of one or several standard density object(s) (emas),
- a step 10 for return transport of the object (100) as far as its location on the turntable (70).

27. Method set forth in claim 26, **characterised in that** the step 4 to determine the significant dimension (x) of the object (100) to be tested consists of the operator inputting a set of input parameters using an interactive module. These parameters include:

- configuration of the turntable (70), in other words the nature of objects that occupy the different locations on the turntable,
- the location occupied by the object (100) on the turntable (70),
- a position ($Z_{measure}$) along the Z direction of the base (26) of the apparatus (2),
- a number P of infrared measurements for each standard dimension object (edim(n)), n = 1, ..., N, where N is the number of standard dimension objects,
- a number Q of infrared measurements for the object (100).

28. Method set forth in claim 27, **characterised in that** the step 4 to determine the significant dimension (x) of the object (100) to be tested also includes the following operation:

a) displacement of a base (26) in the apparatus (2) along the Z direction as far as the position ($Z_{measure}$),
b) displacement of a first infrared assembly (4, 6) along the Y direction, as far as a position ($Y_{measure}$) defined by: Measure = $Y_{OPT}$ + ($X_{edim}$ - $X_{edimAVE}$), where:

$Y_{OPT}$ is the optimum position obtained in calibration step 1,
$X_{edim}$ is the dimension of the standard dimension object (edim) used during the calibration step 1,
$X_{edimAVE}$ is the significant average dimension of all standard dimension objects (edim),

c) measurement of the infrared response RI(p), repeated P times, p = 1, ..., P of N standard dimension objects (edim(n)), n = 1, ..., N, which leads to a set of values RI(n, p),
d) actual calculation of the significant dimension of the object (100).

29. Method set forth in claim 28, **characterised in that** the operation d) to actually calculate the significant dimension (x) of the object (100) is done as follows:

d-1) use of an order 4 polynomial regression of the significant dimensions $X_{edim}(n)$ of each of the N objects with standard dimension (edim), as a function of the average $RI_{edimAVE} = \dfrac{\sum RI(n,p)}{P}$ of infrared responses of said object with standard dimension edim(n), to calculate the coefficients $A_0$, $A_1$, $A_2$, $A_3$, $A_4$ of a relation of the following type:

$$X_{edim}(n) = A_4 \cdot (RI_{edimAVE}(N))^4 + A_3 \cdot (RI_{edimAVE}(n))^3 + A_2 \cdot (RI_{edimAVE}(n))^2 + A_1 \cdot (RI_{edimAVE}(n))^1 + A_0,$$

d-2) measurement of the infrared response RI(q), repeated Q times, q = 1, ..., Q of the object 100 to be tested, and calculate the average $RI = \dfrac{\sum RI(q)}{Q}$ of these infrared responses, and calculate the significant dimension (x) of the object (100) by the following relation:

$$x = A_4 \cdot (RI)^4 + A_3 \cdot (RI)^3 + A_2 \cdot (RI)^2 + A_1 \cdot (RI)^1 + A_0$$

30. Use of the system according to any one of claims 1 to 14 and of the method set forth in any one of claims 15 to 29, for testing objects (100) being manufactured.

31. Use set forth in claim 30, wherein objects (100) are nuclear fuel pellets.

**Patentansprüche**

1. System zur automatischen Bestimmung der Massendichte eines Gegenstandes (100), der zu einer Charge von Gegenständen gehört, **dadurch gekennzeichnet, dass** es aufweist:

   - eine Einheit (2) zur Bestimmung einer signifikanten Abmessung (x) des Gegenstandes (100),
   - eine Einheit (30) zur Bestimmung der Strahlungsstärke (I) eines Photonenstrahls, die durch das Durchlaufen des Gegenstandes (100) gedämpft wird,
   - eine Einheit zur Erfassung, zur Verarbeitung und zur Analyse (200),
   - Einrichtungen zum Transportieren (70, 72, 80, 82, 84, 86, 88) des Gegenstandes (100) zur Einheit (2) zur Bestimmung von dessen signifikanter Abmessung (x) und zur Einheit (30) zur Bestimmung der gedämpften Photonenstrahlungsstärke,
   - erste Einrichtungen zum Anpassen (74, 76, 78) der Position des Gegenstandes (100) relativ zur Einheit zur Bestimmung von dessen signifikanter Abmessung (x), und
   - zweite Einrichtungen zur Anpassung (90, 92, 94, 96, 98) der Position des Gegenstandes (100) relativ zur Einheit (30) zur Bestimmung der gedämpften Photonenstrahlungsstärke,

   weiter **dadurch gekennzeichnet, dass** die ersten Anpassungseinrichtungen und die zweiten Anpassungseinrichtungen fähig sind, den Gegenstand (100) mit einer Genauigkeit in der Größenordnung eines Mikrometers bezüglich einer Trägerplatte (150) zu bewegen, auf der die Bestandteile des Systems installiert sind, und weiter **dadurch gekennzeichnet, dass** die Position des Gegenstandes (100) relativ zur Einheit (30) zur Bestimmung der gedämpften Strahlungsstärke (I) in Abhängigkeit von der signifikanten Abmessung (x) des Gegenstandes (100) angepasst wird.

**2.** System nach Anspruch 1, **dadurch gekennzeichnet, dass** die Erfassungs-, Verarbeitungs- und Analyseeinheit (200) einen Computer (170) aufweist, in den eine dedizierte Software eingebracht ist, die Befehlsabfolgen und Algorithmen zur automatischen Berechnung der Massendichte des Gegenstandes (100) ausführt.

**3.** System nach Anspruch 1 oder 2, **dadurch gekennzeichnet, dass** die Erfassungs-, Verarbeitungs- und Analyseeinheit (200) die relative Variation $\left(\dfrac{\Delta\rho}{\rho}\right)$ der Volumenmasse ($\rho$) des Gegenstandes (100) im Verhältnis zu derjenigen, bekannten, mindestens eines Gegenstandes zur Eichung (emas) liefert, der zur gleichen Charge von Gegenständen (100) gehört.

**4.** System nach einem der Ansprüche 1 bis 3, **dadurch gekennzeichnet, dass** die Einheit (2) zur Bestimmung der signifikanten Abmessung des Gegenstandes (100) aufweist:

- einen ersten Infrarotsatz (4, 6), der aus einer ersten Infrarot-Sendeeinrichtung (4) und einer ersten Infrarot-Empfangseinrichtung (6) besteht,
- einen zweiten Satz (8, 10), der aus einer zweiten Infrarot-Sendeeinrichtung (8) und einer zweiten Infrarot-Empfangseinrichtung (10) besteht,

wobei die zwei Infrarotsätze (4, 6; 8, 10) voneinander um einen bekannten Abstand (d) entfernt angeordnet sind und Infrarotstrahlen aussenden, die zueinander parallel sind, und die signifikante Abmessung (x) des Gegenstandes (100) von der Infrarot-Antwort hergeleitet wird, die erhalten wird, wenn der Gegenstand (100) derart bewegt wird, dass er nacheinander den ersten Infrarotstrahl und den zweiten Infrarotstrahl unterbricht, und zwar entlang einer Richtung im Wesentlichen senkrecht zur Richtung der Achsen (12, 14) der zwei Strahlen, wobei die Infrarot-Antwort dem Anteil (24) des zweiten Strahls entspricht, der vom Gegenstand (100) noch nicht unterbrochen wurde, wenn dieser noch die Hälfte (22) des ersten Strahls unterbricht.

**5.** System nach Anspruch 4, **dadurch gekennzeichnet, dass** weiter die Bestimmungseinheit (2) einen dritten Satz (16, 18) einer photoelektrischen Sende-Empfangseinrichtung aufweist, die, bezogen auf den zweiten Infrarot-Satz (8, 10), vor dem ersten Infrarot-Satz (4, 6) angeordnet ist, und dazu bestimmt ist, vorab ein Anpassen der Strahlungsstärke der zwei Infrarotstrahlen durchzuführen.

**6.** System nach Anspruch 4 oder 5, **dadurch gekennzeichnet, dass** die signifikante Abmessung (x) des Gegenstandes (100) erhalten wird, nachdem veranlasst wurde, dass der Gegenstand QN mal bewegt wird und Q Intrarot-Antworten RI (q) gemessen wurden, für q zwischen 1 und Q, und zwar mittels einer Relation des Typs:

$$x = A_4 \cdot (\text{Mittelwert RI (q)})^4 + A_3 \cdot (\text{Mittelwert RI (q)})^3 +$$
$$A_2 \cdot (\text{Mittelwert RI (q)})^2 + A_1 \cdot (\text{Mittelwert RI (q)})^1 + A_0,$$

wobei:

$A_0$, $A_1$, $A_2$, $A_3$, $A_4$ Koeffizienten sind, die zuvor unter Anwenden ebendieser Relation auf mindestens vier Gegenstände zur Abmessungseichung (edim) erhalten wurden, für die eine Infrarot-Antwort RI (edim) gemessen wird.

**7.** System nach einem der Ansprüche 1 bis 6, **dadurch gekennzeichnet, dass** die Einheit (30) zur Bestimmung der gedämpften Strahlungsstärke eines Photonenstrahls eine Einheit zur Bestimmung mittels Gammaspektrometrie ist, die aufweist:

- einen Satz (32), der aus einer Quelle und einem Kollimator gebildet ist,
- einen Satz (40), der aus einer Erfassungseinrichtung und einem Kollimator gebildet ist,
- eine Kette zur Erfassung und zur Zählung von Gamma-Photonen (48).

**8.** System nach Anspruch 7, **dadurch gekennzeichnet, dass** die Erfassungs- und Zählungskette (48) aufweist:

- eine Erfassungseinrichtung für Germanium hoher Dichte,
- einen Vorverstärker (50),
- ein DSP-(Digitafsignalverarbeitungseinrichtung)-Modul (52),
- ein Hochspannungsmodul (54),
- ein Netzmodul (56),
- einen Datenerfassungscomputer (170),
- einen Kryostaten (60).

9. System nach einem der Ansprüche 1 bis 8, **dadurch gekennzeichnet, dass** die Transporteinrichtungen (70, 72, 80, 82, 84, 86, 88) eine Drehscheibe (70) und einen Schrittmotor (72) zum Antreiben der Scheibe (70) aufweist.

10. System nach einem der Ansprüche 1 bis 9, **dadurch gekennzeichnet, dass** die Transporteinrichtungen einen Handhabungsarm (80) aufweisen.

11. System nach Anspruch 10, **dadurch gekennzeichnet, dass** der Handhabungsarm (80) ein Gelenkarm ist, der mit einer am Ende befindlichen Zange (82) ausgerüstet ist, die dazu bestimmt ist, den Gegenstand (100) zu greifen und abzulegen.

12. System nach einem der Ansprüche 3 bis 11, **dadurch gekennzeichnet, dass** die ersten Anpassungseinrichtungen aufweisen:

   - eine Gleitführung (74) zum Festlegen der Position einer Grundplatte (26) der Einheit (2) zur Bestimmung der signifikanten Abmessung des Gegenstandes entlang einer Richtung X,
   - ein Stellglied (76) zum Bewegen des ersten Infrarot-Satzes (4, 6) bezüglich des zweiten Infrarot-Satzes (8, 10) der Einheit (2) entlang einer Richtung Y senkrecht zur Richtung Y,
   - ein Stellglied (78) zum Bewegen der Grundplatte (26) der Einheit (2) entlang einer Richtung Z senkrecht zur Ebene (X, Y).

13. System nach einem der Ansprüche 1 bis 12, **dadurch gekennzeichnet, dass** die zweiten Anpassungseinrichtungen einen Bestrahlungsträger (90) aufweisen, auf der der Gegenstand (100) installiert ist, und zwar zwischen einer Quelle (32) und einer Erfassungseinrichtung (40) der Einheit (30) zur Bestimmung der gedämpften Strahlungsstärke des den Gegenstand (100) durchquerenden Photonenstrahls.

14. System nach Anspruch 13, **dadurch gekennzeichnet, dass** die zweiten Anpassungseinrichtungen aufweisen:

   - eine Gleitführung (94) zum Festlegen eines Bestrahlungsträgers (90) entlang einer Richtung X,
   - ein Stellglied (96), um, entlang einer Richtung Y senkrecht zur Richtung X, den Bestrahlungsträger (90) zwischen einer Quelle (32) und einer Erfassungseinrichtung (40) der Einheit (30) zur Bestimmung der gedämpften Strahlungsstärke des den Gegenstand (100) durchlaufenden Photonenstrahls zu bewegen,
   - ein Stellglied (98), um, entlang einer Richtung Z senkrecht zur Ebene (X, Y), den Bestrahlungsträger (90) zwischen einer Quelle (32) und einer Erfassungseinrichtung (40) der Einheit (30) zur Bestimmung der gedämpften Strahlungsstärke des den Gegenstand (100) durchlaufenden Photonenstrahls zu bewegen.

15. Verfahren zur Anwendung des Systems zur automatischen Bestimmung der Massendichte eines Gegenstandes (100), der zu einer Charge von Gegenständen gehört, nach einem der Ansprüche 1 bis 14, wobei das System eine Einheit (2) zur Bestimmung einer signifikanten Abmessung (x) eines Gegenstandes (100) und einer Einheit (300) zur Bestimmung der Strahlungsstärke (I) eines Photonenstrahls aufweist, der durch das Durchlaufen des Gegenstandes (100) gedämpft wird, **dadurch gekennzeichnet, dass** es die folgenden Schritte zur Eichung aufweist:

   - einen Schritt 1 zur Eichung der Position der zwei Infrarot-Sätze (4, 6; 8, 10) der Einheit (2) zur Bestimmung der signifikanten Abmessung der Gegenstände (100),
   - einen Schritt 2 zur Eichung der Position eines Bestrahlungsträgers (90) der Einheit (30) zur Bestimmung mittels Gammaspektrometrie der Strahlungsstärke des Photonenstrahls, der durch das Durchlaufen der Gegenstände (100) gedämpft wird,
   - einen Schritt 3 zur Eichung der Messung des Quelle-Erfassungseinrichtung-Satzes (32, 40) der Einheit (30),

   und weiter **dadurch gekennzeichnet, dass** es Schritte zur eigentlichen Bestimmung der signifikanten Abmessung (x) der Gegenstände (100) beinhaltet, die für jeden Gegenstand (100) der Charge von Gegenständen durchgeführt werden.

**16.** Verfahren nach Anspruch 15, **dadurch gekennzeichnet, dass** der Schritt 1 zur Eichung beinhaltet, dass durch die Bedienperson ein Satz von Eingabeparametern mittels eines interaktiven Moduls erfasst werden, wobei darunter sind:

- die Konfiguration von Bestandteilen, die eine Bewegung in Mikrometer-Größenordnung aufweisen, darunter zwei Stellglieder (76, 78),
- die Konfiguration der Drehscheibe (70), d. h. die Beschaffenheit von Gegenständen, die unterschiedliche auf dieser vorgesehene Platzierungen belegen,
- die Platzierung, die von jedem Gegenstand zur Abmessungseichung (edim) auf der Drehscheibe (70) einge-nommen wird,
- eine Position ($Z_{messen}$) einer Grundplatte (26) der Einheit (2) entlang Richtung Z,
- Positionen (Y(1), Y(N)), die ein Bewegungsintervall des ersten Infrarot-Satzes (4, 6) entlang Richtung Y begrenzen,
- einen Schritt (INT) zum Bewegen des ersten Infrarot-Satzes (4, 6) entlang Richtung Y.

**17.** Verfahren nach Anspruch 16, **dadurch gekennzeichnet, dass** der Schritt 1 zur Eichung weiter die folgenden Operationen beinhaltet:

a) Bewegen der Grundplatte (26) entlang Richtung Z bis zur Position ($Z_{messen}$),
b) Winkelbewegung der Drehscheibe (70), um einen Gegenstand zur Abmessungseichung (edim) bis zu seiner Messungs-Initialposition bezüglich der Einheit (2) zu bewegen,
c) Bewegen des ersten Infrarot-Satzes (4, 6) entlang Richtung Y bis zu seiner Startposition (Y(1)),
d) schrittweises Bewegen mittels aufeinanderfolgender inkrementeller Schritte von (INT), des ersten Infrarot-Satzes (4, 6) entlang Richtung Y, wobei er sich dabei vom zweiten Infrarot-Satz (8, 10) entfernt, der an einer Position $Y_{FIX}$ befestigt ist, und zwar zwischen den Positionen (Y(1) und (Y(N)), und gleichzeitiges Bestimmen der Infrarot-Antwort (RI(n)) des Gegenstandes (edim), entsprechend jeder Position (Y(n))
e) Berechnen der optimalen Infrarot-Antwort $RI_{OPT}$,
f) Berechnen der optimalen Position $Y_{OPT}$ des ersten Infrarot-Satzes (4, 6) bezüglich dem zweiten Infrarot-Satz (8, 10).

**18.** Verfahren nach Anspruch 17, **dadurch gekennzeichnet, dass** die Operation d) zum schrittweisen Bewegen die folgenden Unter-Operationen aufweist:

d-1) Winkelbewegung der Drehscheibe (70), um den Gegenstand zur Abmessungseichung (edim) zu seiner Messungsendposition zu transportieren,
d-2) Messen der Infrarot-Antwort (RI(n)) des Gegenstandes zur Abmessungseichung (edim),
d-3) Winkelbewegung der Drehscheibe (70), um den Gegenstand zur Abmessungseichung (edim) zu seiner Messungs-Initialposition zurückzuführen.

**19.** Verfahren nach Anspruch 17 oder 18, **dadurch gekennzeichnet, dass** die optimale Infrarot-Antwort ($RI_{OPT}$) mittels folgender Relation erhalten wird:

$$RI_{OPT} = \frac{RI_{MAX} - RI_{MIN}}{2}$$

wobei: $RI_{MIN}$ der Wert der minimalen Sättigung der Infrarot-Antwort ist,
und: $RI_{MAX}$ der Wert der maximalen Sättigung der Infrarot-Antwort ist.

**20.** Verfahren nach einem der Ansprüche 17 bis 19, **dadurch gekennzeichnet, dass** die Operation f) zur Berechnung der optimalen Position $Y_{OPT}$ in folgender Weise erhalten wird:

$$\text{falls} \quad \frac{RI_{OPT} - RI(j)}{RI_{OPT} - RI(k)} < 1 \quad , \text{dann } Y_{OPT} = Y(j)$$

$$\text{falls} \quad \frac{RI_{OPT} - RI(j)}{RI_{OPT} - RI(k)} > 1 \quad , \text{dann } Y_{OPT} = Y(k)$$

wobei RI(j) und RI(k) zwei zuvor berechnete Werte der Infrarot-Antwort sind, zwischen denen sich die gesuchte optimale Antwort $RI_{OPT}$ befindet, die jeweils zwei Positionen Y(j) und Y(k) des ersten Infrarot-Satzes (4, 6) entsprechen.

**21.** Verfahren nach einem der Ansprüche 15 bis 20, **dadurch gekennzeichnet, dass** der Schritt 2 zur Eichung beinhaltet, dass durch die Bedienperson ein Satz von Eingabeparametern mittels eines interaktiven Moduls erfasst wird, wobei darunter sind:

- die Konfiguration von Bestandteilen, die eine Bewegung in Mikrometer-Größenordnung aufweisen, darunter zwei Stellglieder (96, 98),
- die Konfiguration der Drehscheibe (70), d. h. die Beschaffenheit von Gegenständen, die unterschiedliche Platzierungen auf dieser belegen,
- die Platzierung, die von jedem Gegenstand zur Massendichteeichung (emas) auf der Drehscheibe (70) eingenommen wird,
- eine Messungsdauer oder Zählungszeit,
- Positionen Z(1) und Z(N), die ein Bewegungsintervall eines Bestrahlungsträgers (90) entlang Richtung Z begrenzen,
- eine Anzahl (M) von Messungen der Photonenstrahlungsstärke, die durch das Durchlaufen eines jeden Gegenstandes zur Massendichteeichung gedämpft wird, für jede Position Z(i), die vom Bestrahlungsträger eingenommen wird, für i = 1, ... ,N.

**22.** Verfahren nach Anspruch 21, **dadurch gekennzeichnet, dass** der Schritt 2 zur Eichung weiter die folgenden Operationen beinhaltet:

a) Bestimmen der signifikanten Abmessung ($X_{mas}$) eines jeden Gegenstandes zur Massendichteeichung (emas),
b) Winkelbewegung der Drehscheibe (70) um einen Winkel (A), um den Gegenstand zur Massendichteeichung (emas) in eine Zwischenposition zu bewegen, bei welcher er durch einen Greifarm (80) gegriffen wird,
c) Positionieren des Gegenstandes (emas) auf einem Bestrahlungsträger (90),
d) eigentliches Anpassen der Position des Bestrahlungsträgers (90) bezüglich einer Quelle (32) und einer zugehörigen Erfassungseinrichtung (40),
e) Rücktransport des Gegenstandes zur Massendichteeichung (emas) auf die Drehscheibe (70) mittels einer Operation umgekehrt zur Operation c).

**23.** Verfahren nach Anspruch 22, **dadurch gekennzeichnet, dass** die Operation c) zum Positionieren des Gegenstandes (emas) auf dem Bestrahlungsträger (90) die folgenden Unter-Operationen beinhaltet:

c-1) Bewegen des Bestrahlungsträgers (90) entlang Richtung Z und nach unten,
c-2) Bewegen des Greifarms (80) aus seiner Warteposition bis in die Vertikale der Zwischenposition des Gegenstandes (emas),
c-3) Ergreifen, mittels des Greifarms (80), des Gegenstandes (emas), und Transport von diesem bis in die Vertikale einer Oberseite (92) des Bestrahlungsträgers (90),
c-4) Bewegen des Bestrahlungsträgers (90) bis in die Position Z(1), entlang Richtung Z und nach oben,
c-5) Absetzen des Gegenstandes (emas) auf der Oberseite (92) des Bestrahlungsträgers (90), mittels des Handhabungsarms (80),
c-6) Rückbewegen des Handhabungsarms (80) bis in seine Warteposition,
c-7) Zur-Anlage-Bringen, entlang Richtung Y, des Gegenstandes (emas) auf der Oberseite (92).

**24.** Verfahren nach Anspruch 22 oder 23, **dadurch gekennzeichnet, dass** die Operation d) des eigentlichen Anpassens der Position des Bestrahlungsträgers (90) bezüglich der Quelle (32) und der zugehörigen Erfassungseinrichtung (40) die folgenden Unter-Operationen beinhaltet:

d-1) schrittweises Bewegen des Bestrahlungsträgers (90) entlang Richtung Z zwischen zwei vorbestimmten Positionen Z(1) und Z(N),
d-2) für jede Position Z(i), i = 1, ... , N, Bestrahlen des Gegenstandes zur Massendichteeichung (emas) mittels des Photonenstrahls, und zwar M mal, was zu einem Satz von Werten einer gedämpften Strahlungsstärke I(i, j) führt, wobei i = 1, ..., N die Anzahl von aufeinanderfolgenden Positionen Z(i) repräsentiert, die vom Bestrahlungsträger (90) eingenommen werden, und j = 1, ... , M die Anzahl von Bestrahlungen repräsentiert, die bei jeder Position Z(i) durchgeführt werden,
d-3) Berechnen der optimalen Position ($Z_{OPT}$) des Bestrahlungsträgers (90) ausgehend von einer Polynominalregression vierter Ordnung der Positionen Z(i) bezüglich den gedämpften Strahlungsstärken I(i, j), wobei diese Polynominalregression vierter Ordnung vorbestimmt und integriert ist, als Richtwert einer Erfassungs-, Verarbeitungs- und Analyseeinheit (200).

**25.** Verfahren nach einem der Ansprüche 15 bis 24, **dadurch gekennzeichnet, dass** der Schritt 3 zum Eichen der Einheit 30 zur Bestimmung mittels Gammaspektrometrie die folgenden automatisierten Operationen beinhaltet:

a) Messen der Photonenstrahlungsstärke ($I_{emas}$), die durch das Durchlaufen eines Gegenstandes zur Massendichteeichung (emas) gedämpft wird.
b) Berechnen des Massendämpfungskoeffizienten ($\mu$m) des Gegenstandes zur Massendichteeichung, mittels

der Relation: $\rho_{emas} = -\dfrac{1}{\mu_m \, x_{emas}} \cdot L_n \dfrac{I_{emas}}{I_0}$

**26.** Verfahren nach einem der Ansprüche 15 bis 25, **dadurch gekennzeichnet, dass** die Schritte zum eigentlichen Bestimmen aufweisen:

- einen Schritt 4 zum Bestimmen der signifikanten Abmessung (x) des zu prüfenden Gegenstandes (100),
- einen Schritt 5 zum Transportieren des Gegenstandes (100) zu einem Bestrahlungträger (90),
- einen Schritt 6 zum Anpassen der Position des Gegenstandes (100) mittels Anpassen der Position des Bestrahlungsträgers (90) bezüglich einer Quelle (32) und einer zugehörigen Erfassungseinrichtung (40),
- einen Schritt 7 zum Bestimmen der gedämpften Strahlungsstärke (I) des Photonenstrahls, der den Gegenstand (100) durchlaufen hat,
- einen Schritt 8 zum Erfassen, Verarbeiten und Analysieren des erhaltenen Spektrums,

- einen Schritt 9 zum Bestimmen der relativen Variation $\left( \dfrac{\Delta\rho}{\rho} \right)$ der Massendichte ($\rho$) des Gegenstandes (100)

bezüglich derjenigen eines oder mehrerer Gegenstände zur Massendichteeichung (emas),
- einen Schritt 10 zum Rücktransport des Gegenstandes (100) bis zu seiner Platzierung auf der Drehscheibe (70).

**27.** Verfahren nach Anspruch 26, **dadurch gekennzeichnet, dass** der Schritt 4 zum Bestimmen der signifikanten Abmessung (x) des zu prüfenden Gegenstandes (100) beinhaltet, dass von der Bedienperson ein Satz von Eingabeparametern mittels eines interaktiven Moduls erfasst wird, wobei darunter sind:

- die Konfiguration einer Drehscheibe (70), d. h. die Beschaffenheit der Gegenstände, die unterschiedliche Platzierungen auf dieser belegen,
- die Platzierung, die vom Gegenstand (100) auf der Drehscheibe (70) eingenommen wird,
- eine Position ($Z_{messen}$) entlang Richtung Z der Grundplatte (26) der Einheit (2),
- eine Anzahl P von Infrarot-Messungen für jeden Gegenstand zur Abmessungseichung (edim(n)), n = 1, ... , N, wobei N die Anzahl von Gegenständen zur Abmessungseichung ist,
- eine Anzahl Q von Infrarot-Messungen für den Gegenstand (100).

**28.** Verfahren nach Anspruch 27, **dadurch gekennzeichnet, dass** der Schritt 4 zum Bestimmen der signifikanten Abmessung (x) des zu prüfenden Gegenstandes (100) weiter die folgenden Operationen beinhaltet:

a) Bewegen einer Grundplatte (26) der Einheit (2) entlang Richtung Z bis zur Position ($Z_{messen}$),
b) Bewegen eines ersten Infrarot-Satzes (4, 6) entlang Richtung Y bis zu einer Position ($Y_{messen}$), die definiert ist durch: $Y_{messen} = Y_{OPT} + (X_{edim} - X_{edimMOY})$, wobei:

$Y_{OPT}$ die optimale Position ist, die aus Eichungsschritt 1 hervorgeht,
$X_{edim}$ die Abmessung des Gegenstandes zur Abmessungseichung (edim) ist, die während Eichungsschritt 1 verwendet wird,
$X_{edimMOY}$ der Mittelwert der signifikanten Abmessung aller Gegenstände (edim) zur Abmessungseichung ist,

c) Messen, und zwar P mal wiederholt, der Infrarot-Antwort RI (p), p = 1, ... , P der N Gegenstände zur Abmessungseichung (edim (n)), n = 1, ... , N, was zu einem Satz von Werten RI (n, p) führt,
d) eigentliches Berechnen der signifikanten Abmessung des Gegenstandes (100).

29. Verfahren nach Anspruch 28, **dadurch gekennzeichnet, dass** die Operation d) des eigentlichen Berechnens der signifikanten Abmessung (x) des Gegenstandes (100) in folgender Weise durchgeführt wird:

d-1) Durchführen einer Polynominalregression vierter Ordnung der signifikanten Abmessungen $X_{edim}(n)$ eines jeden der N Gegenstände zur Abmessungseichung edim(n), in Funktion des Mittelwertes der Infrarot-Antworten

$$RI_{edimMOY} = \frac{\sum RI(n, p)}{P}$$ des Gegenstandes zur Abmessungseichung edim(n), um die Koeffizienten $A_0$,

$A_1$, $A_2$, $A_3$, $A_4$ einer Relation folgenden Typs zu berechnen:

$$x_{edim}(n) = A_4 \cdot (RI_{edimMOY}(n))^4 + A_3 \cdot (RI_{edimMOY}(n))^3 +$$
$$A_2 \cdot (RI_{edimMOY}(n))^2 + A_1 \cdot (RI_{edimMOY}(n))^1 + A_0 \quad ,$$

d-2) Messen, und zwar Q mal wiederholt, der Infrarot-Antwort RI(q), q = 1, ... , Q des zu prüfenden Gegenstandes (100), Berechnen des Mittelwertes $RI = \frac{\sum RI(q)}{Q}$ dieser Infrarot-Antworten, und Berechnen der signifikanten Abmessung (x) des Gegenstandes (100) mittels der Beziehung:

$$x = A_4 \cdot (RI)^4 + A_3 \cdot (RI)^3 + A_2 \cdot (RI)^2 + A_1 \cdot (RI)^1 + A_0$$

30. Anwendung des Systems nach einem der Ansprüche 1 bis 14 und des Verfahrens nach einem der Ansprüche 15 bis 29, zum Prüfen von Gegenständen (100) im Verlauf der Herstellung.

31. Anwendung nach Anspruch 30, bei der die Gegenstände (100) Nuklearbrennstofftabletten sind.

FIG. 1

EP 1 733 205 B1

FIG. 2

EP 1 733 205 B1

FIG. 3

FIG. 4

FIG. 5

38

32

34

36

38

**FIG. 6**

40

42

44

46

**FIG. 7**

FIG. 8

FIG. 15

ETALONNAGES PREALABLES

Etape 1

étalonnage de position des deux ensembles infrarouges de l'unité de détermination de la dimension significative de l'objet

Etape 2

étalonnage de position du support d'irradiation de l'unité de détermination de l'intensité du faisceau photonique atténuée par la traversée de l'objet

Etape 3

étalonnage de mesure de l'ensemble source-détecteur de l'unité de détermination de l'intensité du faisceau photonique atténuée par la traversée de l'objet

# FIG. 9A

DETERMINATION PROPREMENT DITE
DE LA VARIATION RELATIVE DE MASSE VOLUMIQUE

Etape 4

détermination de la dimension significative de l'objet à contrôler

Etape 5

transport de l'objet vers le support d'irradiation

Etape 6

ajustement de la position de l'objet par ajustement de la position du support d'irradiation par rapport à une source et un détecteur associé

Etape 7

détermination de l'intensité atténuée du faisceau photonique transmis à travers l'objet

Etape 8

acquisition, traitement et analyse du spectre obtenu

Etape 9

détermination de la variation relative de masse volumique de l'objet par rapport à celle d'un ou plusieurs objet(s) étalon(s) en masse volumique

Etape 10

transport retour de l'objet jusqu'à son emplacement sur le plateau tournant

# FIG. 9B

| Etape 1 |
|---|

| saisie manuelle des paramètres d'entrée |
|---|

séquence d'opérations automatisées :

a) déplacement du socle suivant la direction Z jusqu'à la position $Z_{mesure}$

b) déplacement angulaire du plateau tournant pour amener l'objet edim à sa position initiale de mesure

c) déplacement du premier ensemble infrarouge suivant la direction Y jusqu'à sa position de départ Y(1)

d) déplacement du premier ensemble infrarouge suivant la direction Y, par incréments de INT, et détermination simultanée de la réponse infrarouge RI(n) de l'objet edim, correspondant à chaque position Y(n), pour n = 1, ..., N

e) calcul de la réponse infrarouge optimale

$$RI_{OPT} \frac{RI_{MAX} - RI_{MIN}}{2}$$

f) calcul de la position optimale $Y_{OPT}$ du premier ensemble infrarouge par rapport au deuxième ensemble infrarouge, et de l'écartement optimal d entre les deux ensembles infrarouges

# FIG. 10

Etape 2

saisie manuelle des paramètres d'entrée

séquence d'opérations automatisées :

a) mesure de la dimension significative $x_{emas}$ de l'objet étalon en masse volumique

b) déplacement angulaire du plateau tournant, pour amener l'objet étalon en masse volumique emas en une position intermédiaire où il est saisi par le bras de préhension,

c) positionnement de l'objet emas sur le support d'irradiation

d) ajustement proprement dit de la position du support d'irradiation par rapport à l'ensemble source-détecteur :

d-1) déplacement progressif du support d'irradiation suivant la direction Z entre les positions $Z(1)$ et $Z(N)$ prédéterminées

d-2) pour chaque position $Z(i)$, irradiation M fois de l'objet emas par le faisceau photonique, et obtention des valeurs d'intensité atténuée $I(i, j)$

$i = 1, ..., N$ = nombre de positions $Z(i)$ entre $Z(1)$ et $Z(N)$

$j = 1, ..., M$ = nombre d'irradiations pour chaque position $Z(i)$

d-3) calcul de la position optimale $Z_{OPT}$ du support d'irradiation à partir des positions $Z(i)$ et des intensités atténuées $I(i, j)$

e) transport retour de l'objet emas sur le plateau tournant

# FIG. 11

| Etape 3 |
| --- |

a) mesure de l'intensité photonique $I_{emas}$ atténuée par la traversée d'un objet étalon en masse volumique emas pris comme référence

b) calcul du coefficient massique d'atténuation $\mu_m$ de l'objet étalon en masse volumique par la relation :

$$\rho_{emas} = -\frac{1}{\mu_m X_{emas}} \cdot Ln \frac{I_{emas}}{I_0}$$

# FIG. 12

| Etape 4 |
|---|

| saisie manuelles des paramètres d'entrée |
|---|

séquence d'opérations automatisées :

a) déplacement du socle suivant la direction Z jusqu'à la position $Z_{mesure}$

b) déplacement du premier ensemble infrarouge suivant la direction Y, jusqu'à la position $Y_{mesure} = Y_{OPT} + (x_{edim} - x_{edimMOY})$

c) mesure répétée P fois de la réponse infrarouge RI(p), p = 1, ..., P des N objets étalons en dimension edim(n), n = 1, ... N, conduisant à un ensemble de valeurs RI(n, p),

d) calcul proprement dit de la dimension significative x de l'objet 100 :

d-1) calcul de la moyenne $RI_{edimMOY}$ des P réponses infrarouges de chacun desdits objets edim(n) dont les dimensions significatives $x_{edim}(n)$ sont connues, puis calcul des coefficients $A_0$, $A_1$, $A_2$, $A_3$, $A_4$ de la relation

$x_{edim}(n) = A_4 . (RI_{edimMOY}(n))^4 + A_3 . (RI_{edimMOY}(n))^3 + A_2 . (RI_{edimMOY}(n))^2 + A_1 . (RI_{edimMOY}(n))^1 + A_0$

d-2) mesure de la réponse infrarouge RI(q), q = 1, ..., Q de l'objet 100, puis calcul de la moyenne des RI(q), et calcul de la dimension significative x de l'objet 100 par la relation :

$x = A_4 . (RI)^4 + A_3 . (RI)^3 + A_2 . (RI)^2 + A_1 . (RI)^1 + A_0$

## FIG. 13

Etape 9

calcul automatisé de la variation relative $\dfrac{\Delta\rho}{\rho}$
de masse volumique de l'objet 100 par rapport à celle d'un ou plusieurs objet(s) étalon(s) en masse volumique emas à partir de la relation:

$$\frac{\Delta\rho}{\rho} = \frac{x_{emas}}{x}\left[1 - \frac{L_n\dfrac{I}{I_{emas}}}{\mu_m\rho_{emas}x_{emas}}\right]$$

# FIG. 14

**RÉFÉRENCES CITÉES DANS LA DESCRIPTION**

**Littérature non-brevet citée dans la description**

- **Been, K.** Nondestructive Soil Bulk Density Measurement by X-ray Attenuation. *Geotechnical Testing Journal, GTJODJ,* Décembre 1981, vol. 4 (4), 169-176
- **Tan, S.-A. ; Fwa, T. -F.** Nondestrcutive Density Measurements of Cylindrical Specimens by Gamma-Ray Attenuation. *Journal of Testing Evaluation, JTEVA,* Mars 1991, vol. 19 (2), 155-160
- **Tan, S. -A. ; Fwa, T. -F.** Density Measurements of Cylindrical Specimens within a Mold by Gamma-Rays. *Journal of Testing Evaluation, JTEVA,* Juillet 1993, vol. 21 (4), 296-301